(19) Europäisches Patentamt
European Patent Office
Office européen des brevets

(11) Publication number: **0 042 622**
**B1**

(12) # EUROPEAN PATENT SPECIFICATION

(45) Date of publication of patent specification: **19.08.87**

(21) Application number: **81104864.4**

(22) Date of filing: **23.06.81**

(51) Int. Cl.$^4$: **C 07 D 519/00,**
C 07 D 491/22, C 07 F 9/65,
C 07 F 5/04, C 08 K 5/00 //
(C07D491/22, 319:00, 319:00,
221:00, 221:00)

(54) **Spiro2,2,6,6-tetramethyl-piperidine-4,2′-(1′,3′-dioxo(ol)anr-derivatives and stabilizers for synthetic polymers.**

(30) Priority: **23.06.80 JP 84714/80**

(43) Date of publication of application:
**30.12.81 Bulletin 81/52**

(45) Publication of the grant of the patent:
**19.08.87 Bulletin 87/34**

(84) Designated Contracting States:
**AT BE CH DE FR GB IT LI NL SE**

(56) References cited:
**US-A-4 016 168**
**US-A-4 128 608**
**US-A-4 173 599**

(73) Proprietor: **ADEKA ARGUS CHEMICAL CO., Ltd.**
**1498 Shirahata, Urawa City**
**Saitama Prefecture (JP)**

(72) Inventor: **Kubota, Naohiro**
**404-1 Ageomura**
**Ageo City Saitama Prefecture (JP)**
Inventor: **Shibata, Toshihiro**
**136-49-3-104 Nara-cho**
**Omiya City Saitama Prefecture (JP)**
Inventor: **Sugibuchi, Kazuo**
**3-25-10 Nakagawa Adachi-ku**
**Tokyo (JP)**
Inventor: **Nakahara, Yutaka**
**406-71 Minamishimoarai**
**Iwatsuki City Saitama Prefecture (JP)**

(74) Representative: **Weinhold, Peter, Dr. et al**
**Patentanwälte Dr. V. Schmied-Kowarzik Dipl.-Ing. G. Dannenberg Dr. P. Weinhold Dr. D. Gudel Dipl.-Ing. S. Schubert Dr. P. Barz**
**Siegfriedstrasse 8**
**D-8000 München 40 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 042 622 B1

Courier Press, Leamington Spa, England.

**Description**

US—A—4 128 608 provides 2,2,6,6,-tetramethyl-4-piperidyl spiro aliphatic ethers useful as stabilizers for organic polymeric materials, having the general formula:

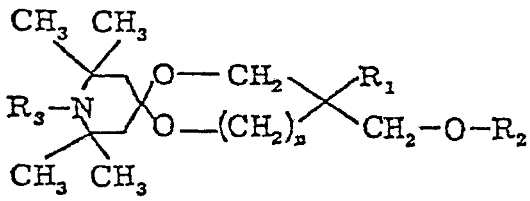

wherein

$R_1$ is selected from the group consisting of hydrogen; lower alkyl and lower hydroxyalkyl groups having 1 or 2 carbon atoms;

$R_2$ is

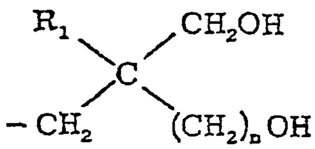

or

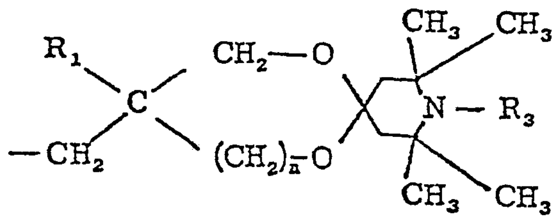

$R_3$ is selected from the group consisting of hydrogen and O•; and

n is 0 or 1.

US—A—4 173 599 provides 2,2,6,6-tetraalkyl-4-piperidyl ketones and ketals useful as stabilizers for organic polymeric materials, having the general formula:

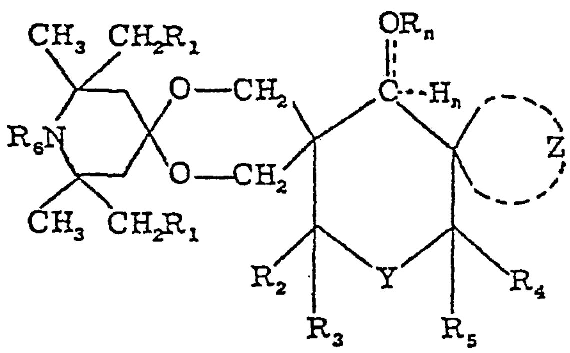

wherein:

n are each 0 or 1 and are each the same;

R is selected from the group consisting of hydrogen; alkyl groups having from 1 to about 18 carbon atoms; and acyl groups

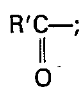

R' being an alkyl group having from 1 to about 18 carbon atoms;

$R_1$, $R_2$, $R_3$, $R_4$ and $R_5$ are selected from the group consisting of hydrogen and alkyl groups having from 1 to about 18 carbon atoms;

$R_6$ is selected from the group consisting of hydrogen and O•; Y is selected from the group consisting of a carbon-to-carbon bond-, —O—; alkylene groups having from 1 to about 3 carbon atoms, and alkylsubstituted alkylene groups, the alkylene group having from 1 to about 3 carbon atoms, the alkyl groups having from 1 to about 6 carbon atoms; and

Z is selected from the group consisting of:

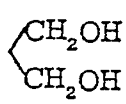

and

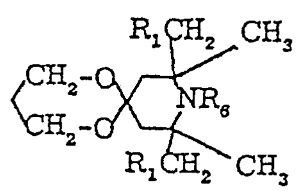

In accordance with the instant invention, spiro[2,2,6,6-tetramethyl piperidine-4,2'-(1',3'-diox(ol)an] derivatives are provided, useful as stabilizers for organic polymeric material, having the general formula:

$$R_1\text{-}[Y\text{-}(CH_2\underset{R_4}{\underset{|}{C}}HO\,\underset{(OR_3)_p}{\underset{|}{X}}OY)_{m}R_2]_n \qquad (I)$$

wherein:

Y is

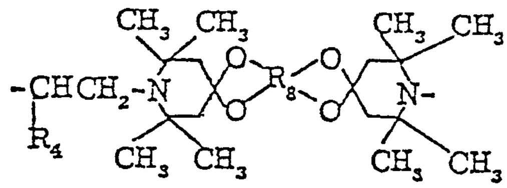

X is selected from the group consisting of alkylene groups having from 1 to 18 carbon atoms; cycloalkylene groups having from 3 to 8 carbon atoms, arylene or aralkylene groups having from 6 to 18 carbon atoms; polyacyl groups

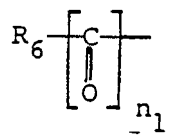

wherein $n_1$ is 2, 3 or 4; polycarbamoyl groups

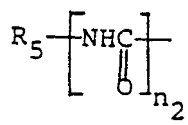

wherein $n_2$ is 2, 3 or 4,

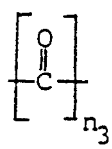

wherein $n_3$ is 1 or 2;

$$-\overset{O}{\overset{\|}{C}}OR_{11}O\overset{O}{\overset{\|}{C}}-;$$

the residues of the polyhydric compounds diethylene glycol, triethylene glycol, thiodiethylene glycol, 1,4-cyclohexanedimethanol, hydrogenated Bisphenol—A and tris(2-hydroxyethyl)isocyanurate; and di- and trivalent oxyacid groups derived from phosphorus-containing oxyacids, silicic acid or boric acid and esters thereof as well as dimers thereof joined by polyhydric alcohols and phenols;

$R_1$ is selected from the group consisting of hydroxy,

$$R_7O\overset{O}{\overset{\|}{C}}-O-,\quad (R_7O\overset{O}{\overset{\|}{C}})_q R_{12}(\overset{O}{\overset{\|}{C}}-O)_n,\quad (R_7O)_{2-n}-\underset{R_{13}}{\underset{|}{\overset{(O)_r}{\overset{\|}{P}}}}-(O)_n \text{ and } (R_7O)_{3-n}-B-(O)_n\ ;$$

$R_2$ is selected from the group consisting of hydrogen and $-CH_2\underset{R_4}{\underset{|}{C}}HOR_{10}$;

$R_3$ is selected from the group consisting of alkyl groups having from 1 to 18 carbon atoms; cycloalkyl groups having from 3 to 8 carbon atoms; aryl or aralkyl groups having from 6 to 18 carbon atoms; and $-X-O-R_1$;

$R_4$ is selected from the group consisting of hydrogen; aliphatic groups having from 1 to 18 carbon atoms; cycloaliphatic groups having from 3 to 8 carbon atoms; and aromatic or araliphatic groups having from 6 to 18 carbon atoms;

$R_5$ is selected from the group consisting of aliphatic groups having from 1 to 18 carbon atoms; cycloaliphatic groups having from 3 to 8 carbon atoms, and aromatic or araliphatic groups having from 6 to 18 carbon atoms; and the residues of lysinediisocyanate, 3-isocyanatomethyl-3,5,5-trimethylcyclohexylisocyanate, bis-(isocyanatomethyl)-cyclohexane, 3-(2'-isocyanatocyclohexyl)propylisocyanate and diphenyletherdisocyanate;

$R_6$ is selected from the group consisting of the residues of saturated or unsaturated aliphatic di-, tri- or tetracarboxylic acids, said residues having up to 18 carbon atoms; saturated or unsaturated cycloaliphatic di-, tri- or tetracarboxylic acids, said residues having up to 8 carbon atoms; aromatic or aryliphatic di-, tri- or tetracarboxylic acids, said residues having up to 18 carbon atoms; and the residues of iminodiacetic acid, thiodipropionic acid, diglycolic acid, thiophene dicarboxylic acid, furane dicarboxylic acid, dicarboxyethylpiperidine and nitrilotriacetic acid;

$R_7$ is selected from the group consisting of the residues of monohydric alcohols having from 1 to 18 carbon atoms and optionally (cyclo)alkyl or halogen substituted phenols having altogether from 6 to 50 carbon atoms;

$R_8$ is selected from the group consisting of

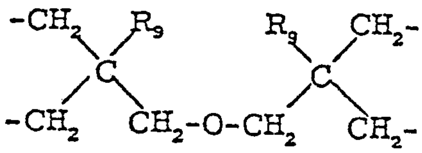 , 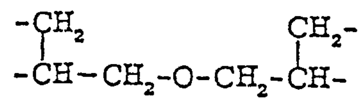 and

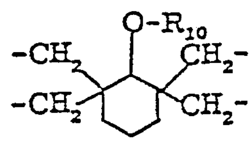

$R_9$ is selected from the group consisting of aliphatic groups having from 1 to 18 carbon atoms, cycloaliphatic groups having from 3 to 8 carbon atoms; and $-CH_2OR_{10}$:

$R_{10}$ is selected from the group consisting of hydrogen,

$$-\overset{\overset{O}{\|}}{C}-R_{12}-(\overset{\overset{O}{\|}}{C}OR_7)_{n+q-l},\quad -\overset{\overset{O}{\|}}{C}OR_7,\quad -\underset{\underset{R_{13}}{|}}{\overset{\overset{(O)_r}{\|}}{P}}-OR_7,\ \text{and}\ -B-(OR_7)_2;$$

$R_{11}$ is selected from the group consisting of alkylene and oxyalkylene groups having from 2 to 10 carbon atoms and from 0 to 5 oxyether groups; cycloalkylene groups having from 3 to 8 carbon atoms; and optionally hydrocarbyl substituted arylene groups having altogether from 6 to 50 carbon atoms, including optionally hydrocarbyl substituted phenylene groups joined by an oxygen or sulfur atom or an optionally hydrocarbyl substituted methylene group; isocyanurate; and the residues of thiodiethylene glycol, 1,4-phenyldimethanol, hydrogenated Bisphenol-A, bis - (2 - methyl - 4 - hydroxy - 5 - t - butylbenzyl) - sulfide, 1,1,3 - tris - (2' - methyl - 4' - hydroxy - 5' - t - butylphenyl) butane, 2,2 - bis(3' - t - butyl - 4' - hydroxyphenyl) - 4 - (3'',5'' - di - t - butyl - 4'' - hydroxyphenyl)butane and 2,2 - bis - (2' - methyl - 5' - t - butyl - 4' - hydroxyphenyl) - 4 - (3'',5'' - di - t - butyl - 4'' - hydroxyphenyl)butane;

$R_{12}$ is the residue of a saturated or unsaturated (cyclo)aliphatic, aromatic or N—, O— or S-heteroaromatic mono-, di- tri- or tetracarboxylic acid, having from 2 to 22 carbon atoms and optionally containing keto or hydroxy groups; or the residue of aminoacetic acid, dodecyl mercaptopropionic acid, 3,5 - di - t - butyl - 4 - hydroxyphenyl - propionic acid, 2,2,6,6 - tetramethyl - piperidine - 4 - carboxylic acid, 3,8,8,10,10 - pentamethyl - 9 - aza - 1,5 - dioxaspiro[5,5]undecane - 3 - carboxylic acid, iminodiacetic acid, thiodipropionic acid, diglycolic acid, dicarboxyethyl piperidine and nitrilotriacetic acid;

$R_{13}$ is selected from the group consisting of hydrogen; alkyl or alkoxy groups having from 1 to 18 carbon atoms; cycloalkyl or cycloalkoxy groups having from 3 to 8 carbon atoms; aromatic, O-aromatic or araliphatic and O-ar-aliphatic groups having from 6 to 18 carbon atoms;

$$R_7-O-\underset{\underset{O}{\|}}{C}-(O)-;$$

monovalent oxyacid groups derived from phosphorus containing acids, silicic acid or boric acid; and (4-hydroxy-3,5-di-tert.-butyl phenyl)-methyl;

m is a number from zero to 10;

n is a number from 1 to 4;

p is zero, 1 or 2;

q is a number from zero to 3; and

r is zero or 1.

In general formula (I) exemplary $R_3$, $R_4$, $R_5$, $R_6$, $R_9$ and $R_{13}$ alkyl(ene) groups include methyl(ene), ethyl(ene), propyl(ene), butyl(ene), pentyl(ene), hexyl(ene), heptyl(ene), octyl(ene), 2-ethylhexyl(ene), isooctyl(ene), nonyl(ene), decyl(ene), undecyl(ene), dodecyl(ene), tridecyl(ene), tetradecyl(ene), hexadecyl(ene), pentadecyl(ene), heptadecyl(ene), octadecyl(ene); exemplary $R_3$, $R_4$, $R_5$, $R_6$, $R_9$ and $R_{13}$ cycloalkyl(ene), groups include cyclobutyl(ene), cyclopropyl(ene), cyclopentyl(ene), cyclohexyl(ene), cyclopropyl(ene), and cyclooctyl(ene); exemplary $R_3$, $R_4$, $R_5$, ($R_6$), $R_9$ and $R_{13}$ aryl(ene) or aralkyl(ene) groups include benzyl(ene), phenylethyl(ene), phenyl(ene), toluyl(ene), xylyl(ene), butylphenyl(ene) and nonylphenyl(ene).

Exemplary $R_7$ groups are the residues of monohydric alcohols having 1 to 18 carbon atoms such as methanol, ethanol, (iso)propanol, butanol, pentanol, (cyclo)hexanol, heptanol, octanol, 2-ethylhexanol, isooctanol, nonanol, decanol, isodecanol, undecanol, lauryl alcohol, tridecanol, myristyl alcohol, pentadecanol, palmityl alcohol, heptadecanol, stearyl alcohol; mono-, di-, and triethylene glycol monoether; benzyl alcohol and phenyl ethanol; and the residues of optionally (cyclo)alkyl or halogen substituted monohydric phenols such as phenol, cresol, 4-t-butylphenol, octylphenol, nonylphenol, 2,6-dimethylphenol, 2-cyclohexylphenol, 2,4-di-t-butylphenol, 2-t-butyl-4-methylphenol, dinonylphenol and chlorphenol.

Exemplary $R_{11}$ groups are the residues of dihydric (cyclo)aliphatic alcohols such as ethylene glycol, 1,2-propanediol, 1,3-propanediol, 1,2-butanediol, 1,3-butanediol, 1,4-butanediol, pentyl glycol, 1,6-hexanediol, 1,10-decanediol, 1,4-cyclohexanediol and 1,4-cyclohexanedimethanol; polyalkylene glycols such as diethylene glycol and triethylene glycol; trihydric aliphatic alcohols such as glycerol, trimethylolmethane and trimethylolethane; optionally hydrocarbyl substituted dihydroxy benzenes such as hydroquinone, 2,5-di-t-butylhydroquinone, 2,3,6-tri-methyl hydroquinone and 2-methylresorcinol; methylene bisphenols, optionally having hydrocarbyl substituents on the phenyl rings and/or the methylene carbon atom such as 4,4'-isopropylidene diphenol (Bisphenol-A), 4,4'-cyclohexylidenediphenol, 4,4'-methylenebisphenol, 2,2'-methylene bis(4-methyl-6-t-butylphenol), 2,2'-methylene bis (4-ethyl-6-t-butylphenol), 2,2'-methylene bis[4-methyl-6-(α-methylcyclohexyl)phenol], 2,2'-n-butylidenebis (4,6-di-methylphenol), bis-1,1-(2'-hydroxy-3'-5'-di-methylphenyl)-3,5,5-trimethylhexane, 2,2'-cyclohexylidene-bis-(4-ethyl-6-t-butylphenol), 2,2'-isopropyl-benzylidene-bis-(4-ethyl-6-t-butylphenol), 4,4'-methylene-bis-(2-methyl-6-t-butylphenol), 4,4'-isopropylidene-bis-(2-phenylethylphenol), 4,4'-n-butylidene-bis-(3-methyl-6-t-butylphenol), 4,4'-cyclohexylidene-bis-(2-t-butylphenol), 4,4'-cyclohexylidenebis-(2-cyclohexylphenol) and 4,4'-benzylidene-bis-(2-t-butyl-5-methylphenol); oxo or thio bisphenols, optionally having hydrocarbyl substituents on the phenyl rings such as 4,4'-oxobis-(3-methyl-6-isopropylphenol), 4,4'-thiobisphenol, 2,2'-thiobis-(4-t-butyl-6-methylphenol), 2,2'-thiobis-(4-methyl-6-t-butylphenol), 2,2'-thiobis(4,6-di-t-butylphenol), 4,4'-thiobis(2-methyl-6-t-butylphenol) and 4,4'-thiobis-(3-methyl-6-t-butylphenol); and tris (2-hydroxyethyl)isocyanurate.

Exemplary $R_{12}$ groups are the residues of saturated or unsaturated (cyclo)aliphatic carboxylic acids such as formic acid, acetic acid, propionic acid, butyric acid, pivalic acid, 2-ethylhexanoic acid, lauric acid, palmitic acid, stearic acid, acrylic acid, crotonic acid, oleic acid; oxalic acid, malonic acid, succinic acid, glutaric acid, adipic acid, sebacic acid, dodecane dicarboxylic acid, eicosane dicarboxylic acid, maleic acid, tetrahydrophthalic acid, endomethylenetetrahydrophthalic acid; tricarballylic acid, butanetricarboxylic acid, butenetricarboxylic acid; ethylenetetracarboxylic acid, ethanetetracarboxylic acid, 1,2,2,3-propanetetracarboxylic acid and 1,1,2,3-propanetetracarboxylic acid; aromatic carboxylic acids such as benzoic acid, toluic acid, 4-t-butylbenzoic acid; phthalic acid, isophthalic acid, terephthalic acid and trimellitic acid; N-, O- or S-heteroaromatic carboxylic acids such as nicotinic acid, isonicotinic acid, thiophene-dicarboxylic acid and furane-dicarboxylic acid; and carboxylic acids as above but additionally containing keto or hydroxy groups such as acetoacetic acid, levulinic acid, pyruvic acid, ketostearic acid, 3,5-di-t-butyl-4-hydroxybenzoic acid, tartaric acid and citric acid.

Examplary X alkylene groups are methylene and the residues of polyhydric alcohols such as ethylene glycol, 1,2-propanediol, 1,3-propanediol, 1,2-butanediol, 1,3-butanediol, 1,4-butanediol, pentyl glycol, 1,6-hexanediol and 1,10-decanediol.

An exemplary X cycloalkylene group is cyclohexylene, e.g. the residue of 1,4-cyclohexanediol.

Exemplary X arylene groups are phenylene and biphenylene.

Examplary X polyacyl groups are derived from saturated and unsaturated (cyclo)aliphatic polycarboxylic acids such as oxalic acid, malonic acid, succinic acid, glutaric acid, adipic acid, sebacic acid, dodecane dicarboxylic acid, eicosane dicarboxylic acid, tartaric acid, maleic acid, tetrahydrophthalic acid, endomethylenetetrahydrophthalic acid, citric acid, tricarballyic acid, butanetricarboxylic acid, butenetricarboxylic acid, ethylenetetracarboxylic acid, ethanetetracarboxylic acid, 1,2,2,3-propanetetracarboxylic acid and 1,1,2,3-propanetetracarboxylic acid; aromatic polycarboxylic acids such as phthalic acid, isophthalic acid, terephthalic acid and trimellitic acid.

Exemplary X polycarbamoyl groups are derived from aliphatic polyisocyanates such as hexamethylenediisocyanate and aromatic or araliphatic polyisocyanates such as phenyldiisocyanate, toluylenediisocyanate and xylylenediisocyanate.

Exemplary monovalent or polyvalent groups derived from oxyacids are the groups derived from phosphorus-containing oxyacids such as phosphoric acid, phosphonic acid, phosphorous acid, phosphonous acid, phosphinic acid and phosphinous acid and derived from silicic acid, boric acid and esters thereof as well as dimers thereof, joined by a polyhydric alcohol or phenol.

Typical compounds having the general formula (I) are shown below:

1.

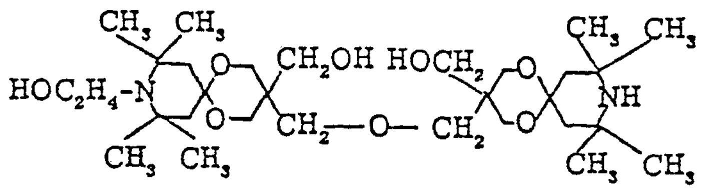

2.

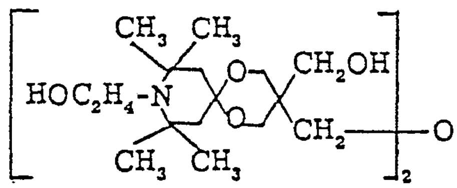

3.

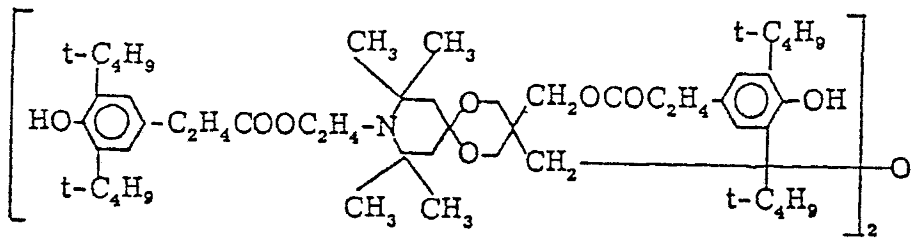

4.

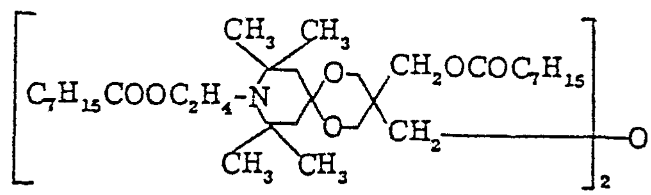

5.

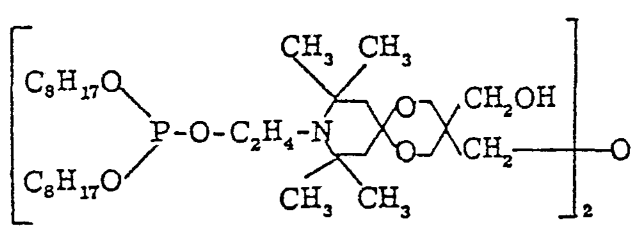

6.

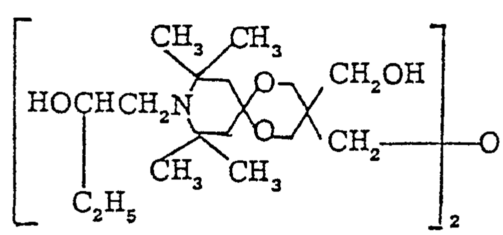

7.

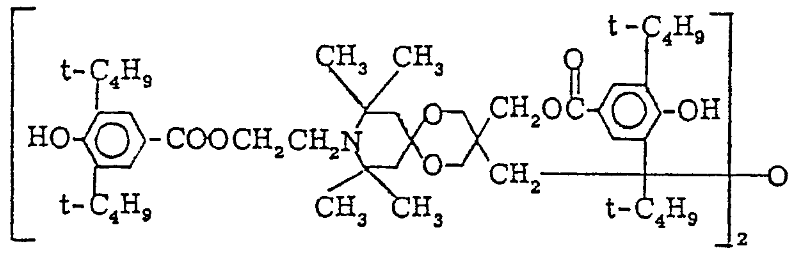

8.

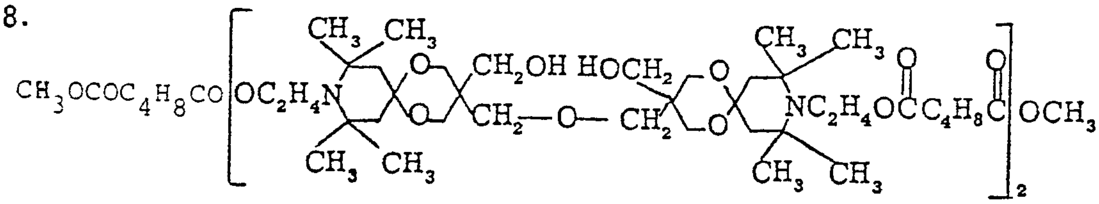

9.

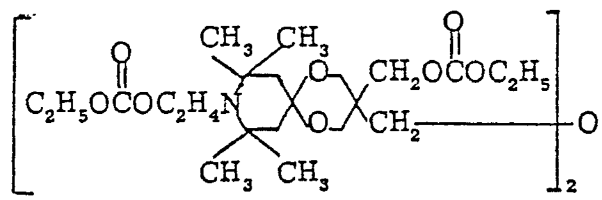

10.

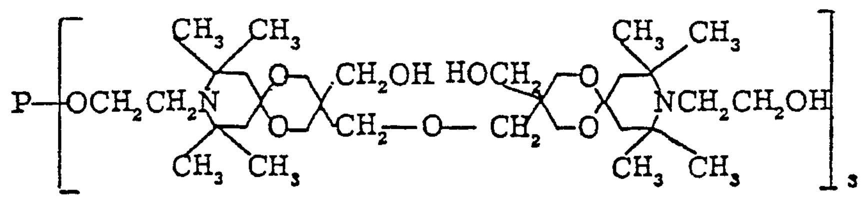

11.

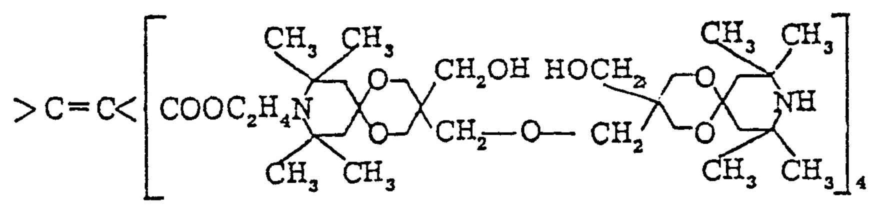

12.

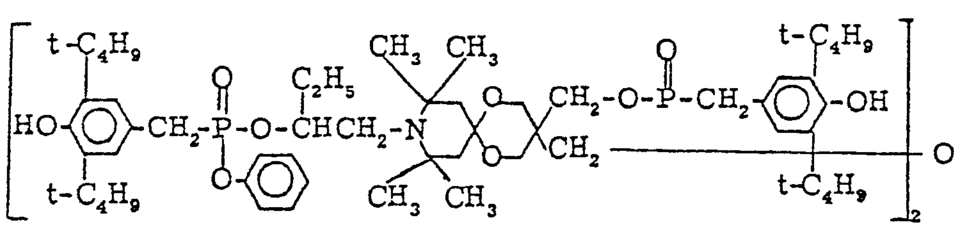

13.

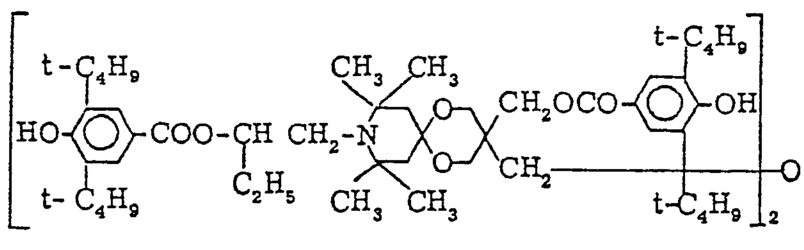

14.

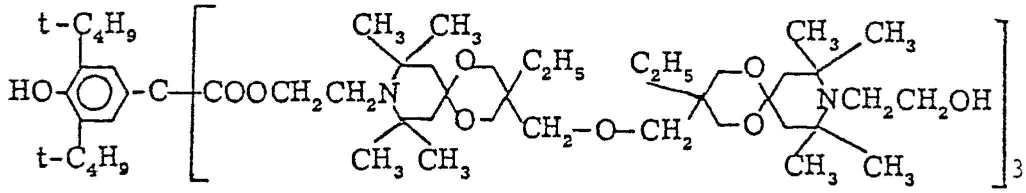

15.

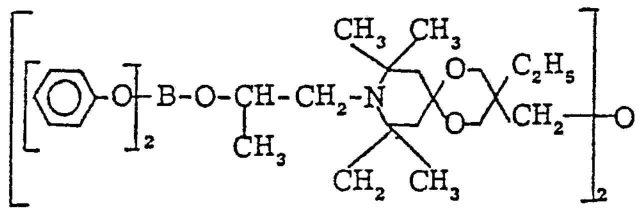

16.

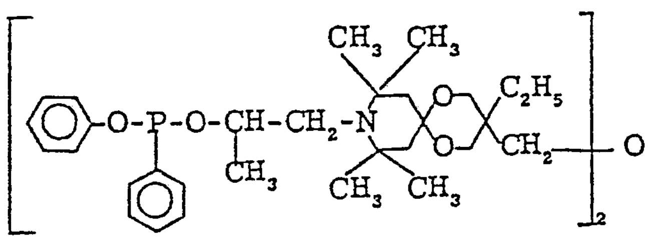

17.

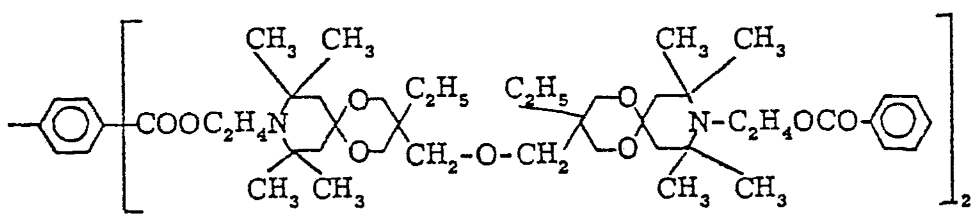

18.

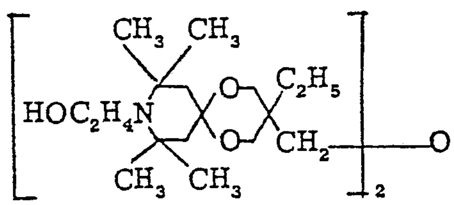

19.

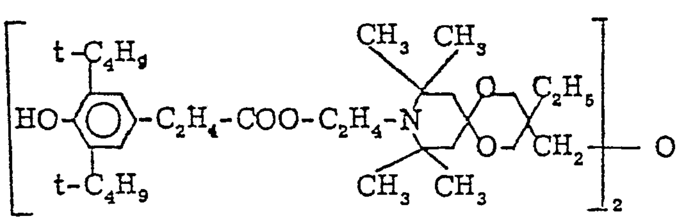

20.

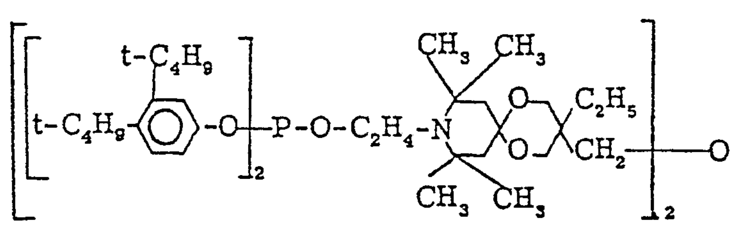

21.

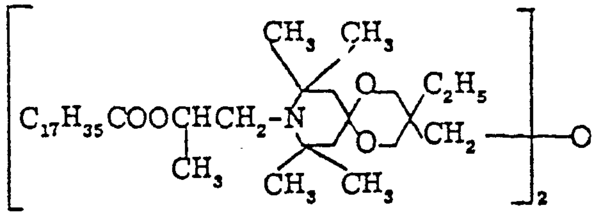

22.

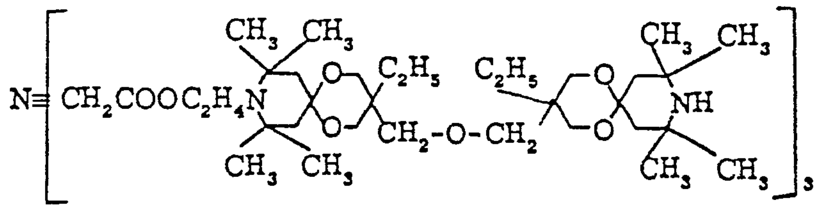

23.

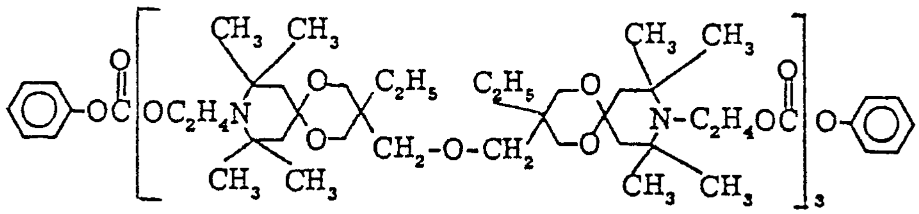

24.

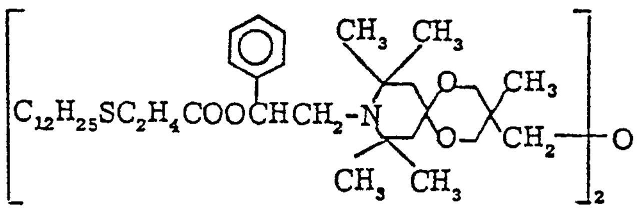

25.

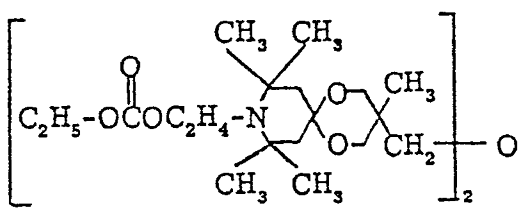

26.

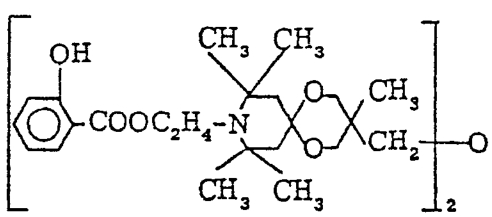

27.

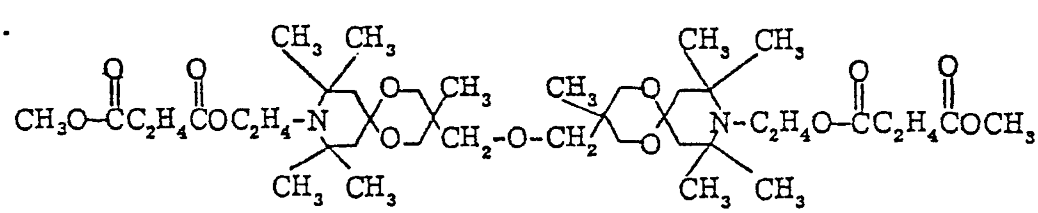

28.

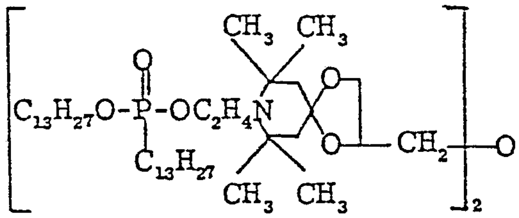

29.

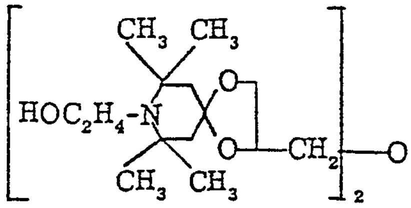

30.

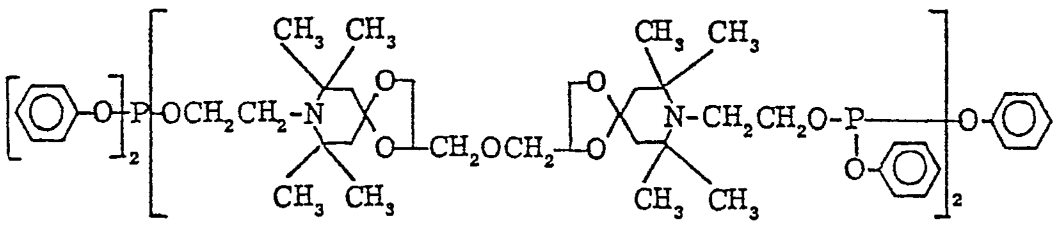

31.

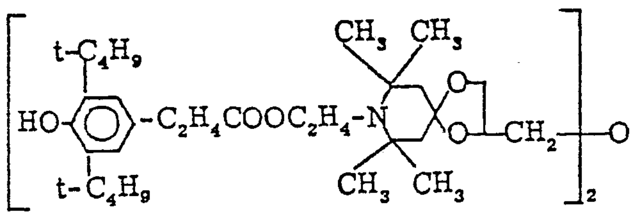

32.

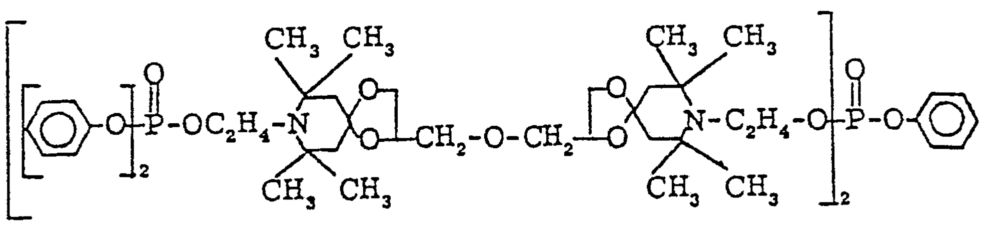

33.

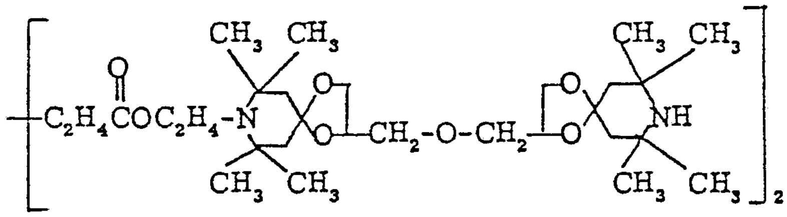

34.

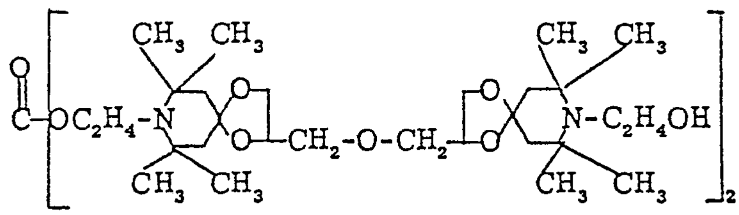

36.

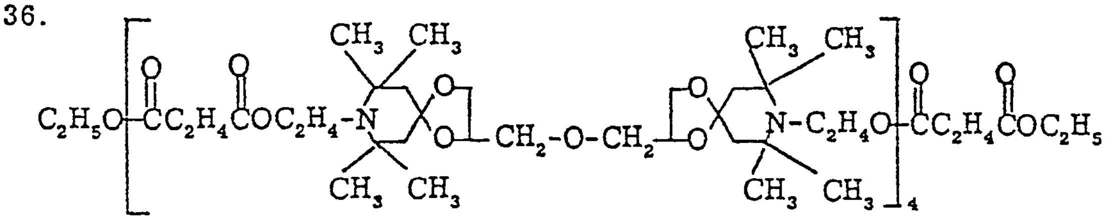

35.

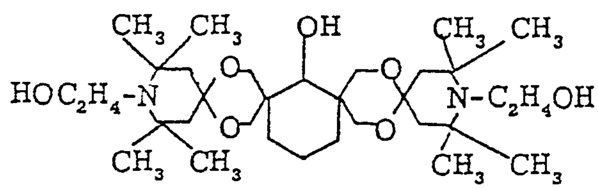

37.

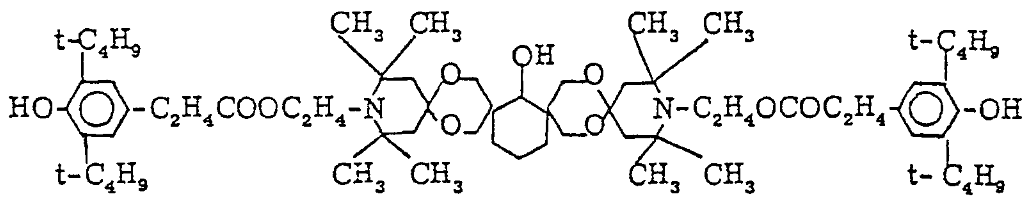

38.

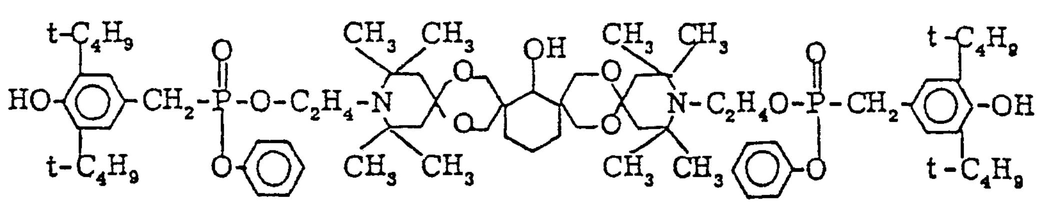

39.

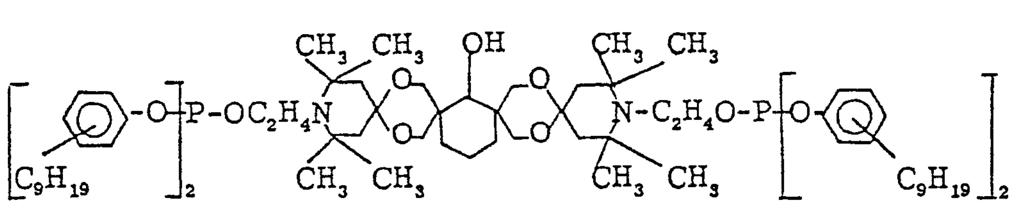

40.

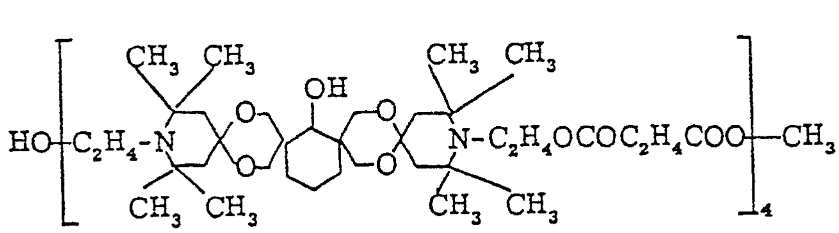

41.

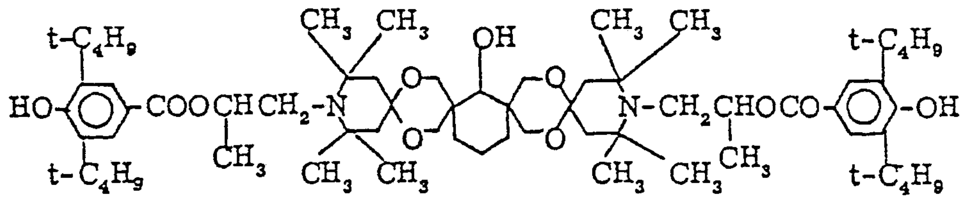

42.

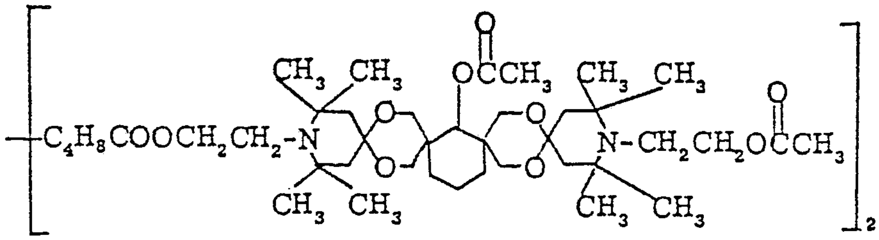

Compounds having the general formula (I) of this invention are readily prepared by the reaction of the base compound:

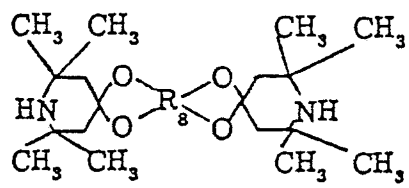

with

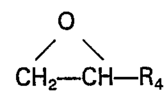

followed by esterification, if necessary.

They also can be prepared by the reaction of

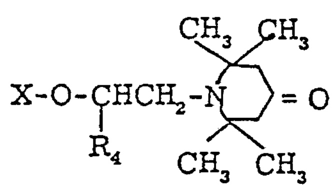

prepared beforehand with

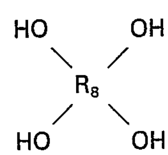

The following Examples illustrate the preparation of the exemplary compounds of the invention noted above:

Example I

Preparation of:

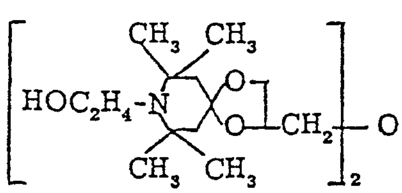

7.5 g of oxabis (8-aza-7,7,9,9-tetramethyl-1,4-dioxaspiro[4,5]-3-decylmethane) was dissolved in a mixture of water 40 ml and ethanol 30 ml, and then ethylene oxide was blown into the mixture at 25°C. The mixture was stirred for 35 hours under reflux. After solvent was distilled off, the product was dissolved in toluene and washed by water, and then the toluene was distilled off.

8.2 g of viscous pale yellow liquid was obtained.

Amine value:

Calculated: 5.30%
Found : 5.41%

Example II

Preparation of:

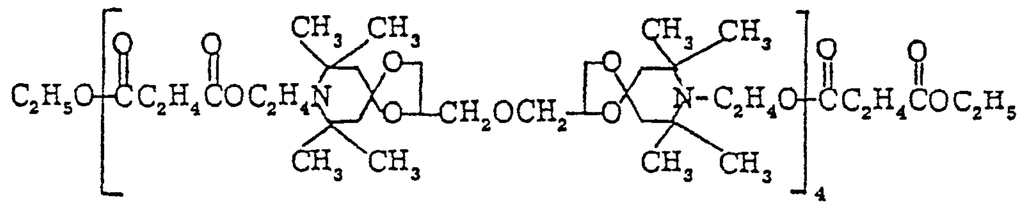

The mixture of the compound obtained in Example I, 2.64 g, diethyl succinate 1.09 g, xylene 20 ml, and lithium amide 0.04 g was reacted for nine hours under reflux. After cooling, the mixture was washed, dried and solvent was distilled off, and 3.0 g of resinous product was obtained. The molecular weight of the product, measured by the vapor pressure method, was about 2,500.

The compounds of the invention are effective stabilizers to enhance the resistance to deterioration due to heat and/or light of synthetic polymeric materials which are susceptible to such degradation, including polyolefins such as low density polyethylene, high density polyethylene, polypropylene, polybutylene, polyisobutylene, polypentylene, and polyisopentylene; polystyrene; polydienes, such as polybutadiene and polyisoprene; and copolymers of colefins and dienes with other ethylenically and acetylenically unsaturated monomers, such as ethylene-propylene copolymers, ethylenebutene copolymers, ethylene-pentene copolymers, ethylene-vinyl acetate copolymers, styrene-butadiene copolymers, acrylonitrilestyrene-butadiene copolymers, synthetic rubbers of all types, such as polychloroprene; polyvinyl halides, including polyvinyl chloride homopolymer; polyvinylidene chloride; and copolymers of vinyl chloride and vinylidene chloride; vinyl chloride and vinyl acetate; vinylidene chloride and vinyl acetate; and other ethylenically unsaturated monomers; polyacetals such as polyoxymethylene and polyoxyethylene; polyesters such as polyethylene glycol-terephthalic acid ester polymers; polyamides such as polyepsiloncaprolactam, polyhexamethylene adipamide and polydecamethylene adipamide; polyurethanes; and epoxy resins.

The synthetic polymer can be in any physical form, including (for example) filaments, yarns, films, sheets, molded articles, latex, and foam.

The compounds of the invention can be used as a stabilizer in an amount within the range from about 0.001 to about 10 parts by weight, preferably from 0.01 to 5 parts by weight, per 100 parts by weight of resin.

The compounds of the invention can also be incorporated in the reaction mixture of monomers of polymerizable components used for preparation of the polymer to be stabilized, in which event the stabilizer of the invention can become a constituent part of the polymer molecule, and exert its stabilizing effect there. Examples are the reaction mixtures for preparation of polyaddition or polycondensation polymers such as polyurethanes and polyesters. In such cases, amounts of compound of the invention within the range from about 0.001 to about 10 parts by weight, preferably from 0.01 to 5 parts by weight, per 100 parts by weight of total monomers of polymerizable components can be used.

The stabilizers of the invention can be employed as the sole stabilizer or, preferably, in combination with other conventional heat and light stabilizers for the particular synthetic polymer.

Thus, for example, in the case of polyvinyl chloride resins, other polyvinyl chloride resin heat stabilizers can be included, including polyvalent metal fatty acid salts such as barium and cadmium salts of the higher fatty acids; organic triphosphites; organotin compounds; hindered phenols; and epoxy compounds.

With polyolefin resins there can be employed fatty acid salts of polyvalent metals, organic phosphites, phenolic and thiophenolic antioxidants, and the higher fatty alcohol esters of thiodipropionic acids, such as, for example, dilauryl thiodipropionate.

With polyamide resin compositions, polyamide stabilizers such as copper salts in combination with iodides and/or phosphorous compounds and salts of divalent manganese can be used.

With synthetic rubbers and acrylonitrile-butadiene-styrene terpolymers, antioxidants such as hindered phenols and bis-phenols, polyvalent metal salts of the higher fatty acids, and organic phosphites can be used.

In addition, other conventional additives for synthetic polymers, such as plasticizers, lubricants, emulsifiers, antistatic agents, flame-proofing agents, pigments and fillers, can be employed.

The following Examples represent preferred embodiments of synthetic resin compositions in accordance with the invention:

Examples 1 to 12

A group of polyvinyl chloride resin compositions was prepared having the following formulation:

| Ingredient | Parts by Weight |
|---|---|
| Polyvinyl chloride | 100 |
| Dioctylphthalate | 48 |
| Epoxidized soybean oil | 2 |
| Tris-nonyl phenyl phosphite | 0.2 |
| Ca stearate | 1.0 |
| Zn stearate | 0.1 |
| Stabilizer as shown in Table I | 0.3 |

This formulation was blended and sheeted off on a two-roll mill to form sheets 1 mm thick. The light resistance of these sheets was then determined by placing strips 1 cm wide in a Weather-O-Meter®, and exposing them to ultraviolet light. The time in hours was then noted for the sheets to develop a noticeable discoloration and/or embrittlement, indicating deterioration due to oxidation in the presence of ultraviolet light.

This test was repeated for the stabilizers in accordance with the invention, having the formulae indicated in Table I, in comparison with four controls, oxabis - (9 - aza - 3 - ethyl - 8,8,10,10 - tetramethyl - 1,5 - dioxaspiro[5,5] - 3 - undecylmethane), bis - (9 - aza - 3 - ethyl - 8,8,10,10 - tetramethyl - 1,5 - dioxaspiro[5,5] - 3 - undecylmethyl) carbonate, oxabis - (9 - aza - 3 - hydroxymethyl - 8,8,10,10 - tetramethyl - 1,5 - dioxaspiro[5,5] - 3 - undecyl methane) and 1 - (3,5 - di - t - butyl - 4 - hydroxyphenylpropionyloxyethyl) - 2,2,6,6 - tetramethyl - 4 - piperidinyl - 3,5 - di - t - butyl - 4 - hydroxyphenylpropionate.

The following results were obtained:

TABLE I

| | Stabilizer | Hours to Failure |
|---|---|---|
| Control 1 | None | 180 |
| Control 2 | Oxabis-(9-aza-3-ethyl-8,8,10,10-tetramethyl-1,5-dioxaspiro [5,5]-3-undecylmethane) | 590 |
| Control 3 | Bis-(9-aza-3-ethyl-8,8,10,10-tetramethyl-1,5-dioxaspiro [5,5]-3-undecylmethyl) carbonate | 260 |
| Control 4 | Oxabis-(9-aza-3-hydroxymethyl-8,8,10,10-tetramethyl-1,5-dioxaspiro [5,5]-3-undecyl methane) | 600 |
| Control 5 | 1-(3,5-di-t-butyl-4-hydroxyphenylpropionyloxyethyl)-2,2,6,6-tetramethyl-4-piperidinyl-3,5-di-t-butyl-4-hydroxyphenylpropionate | 430 |
| Example No. | | |
| 1 | 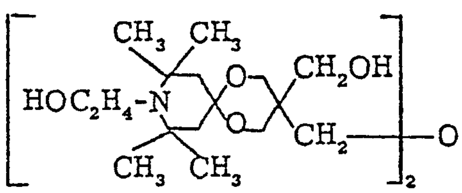 | 750 |
| 2 | 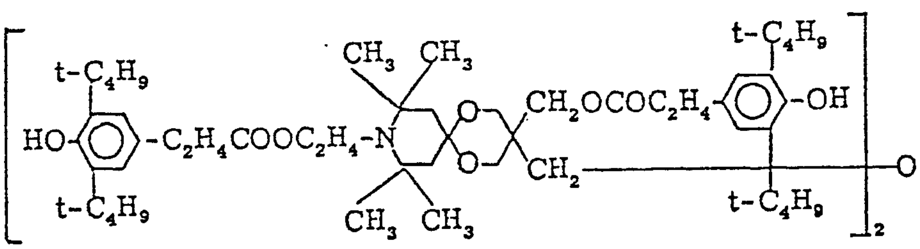 | 820 |
| 3 | 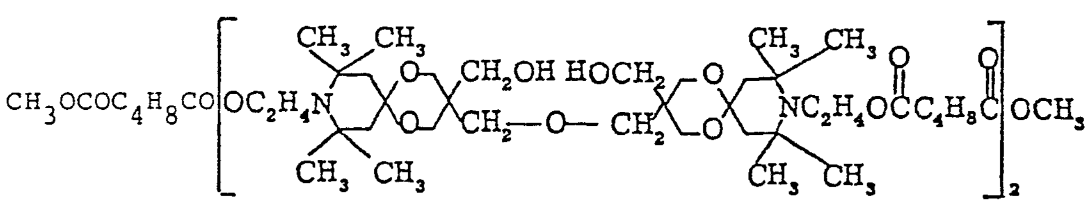 | 840 |

TABLE I (continued)

| Example No. | Stabilizer | Hours to Failure |
|---|---|---|
| 4 | 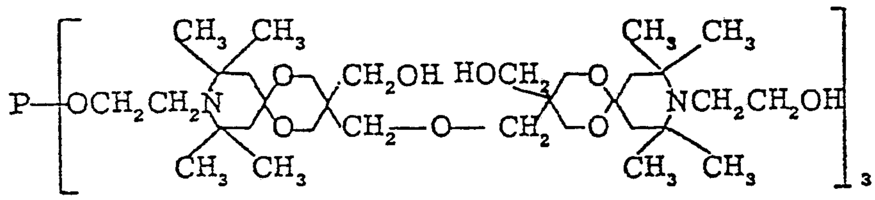 | 790 |
| 5 | 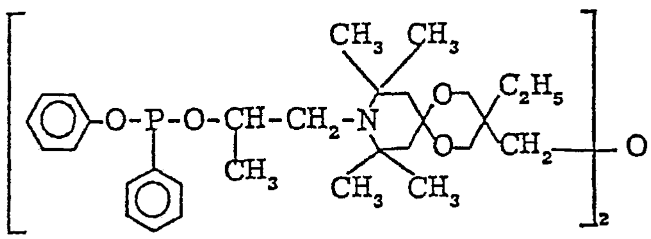 | 810 |
| 6 | 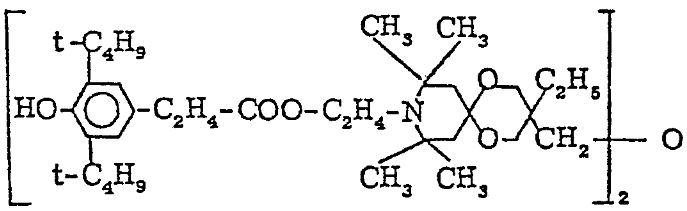 | 820 |
| 7 | 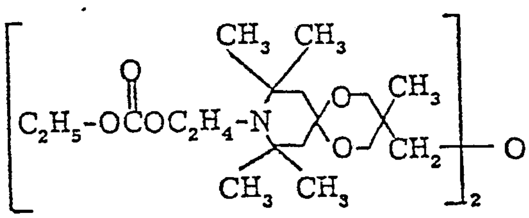 | 780 |
| 8 | 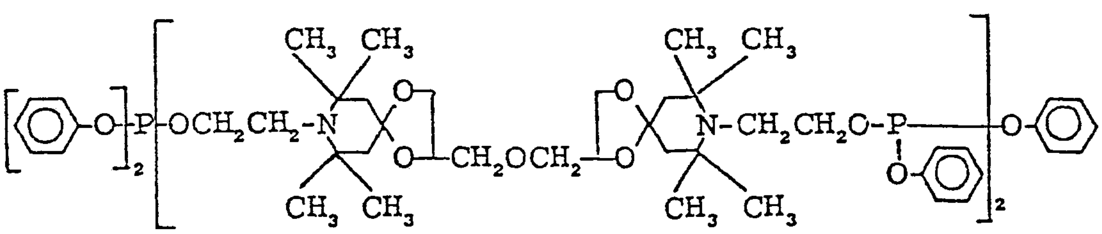 | 810 |

TABLE I (continued)

| Example No. | Stabilizer | Hours to Failure |
|---|---|---|
| 9 | 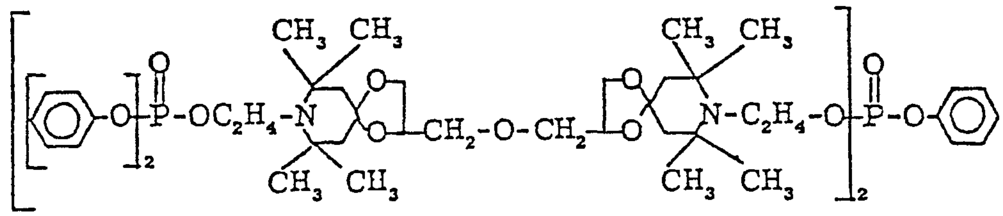 | 780 |
| 10 | 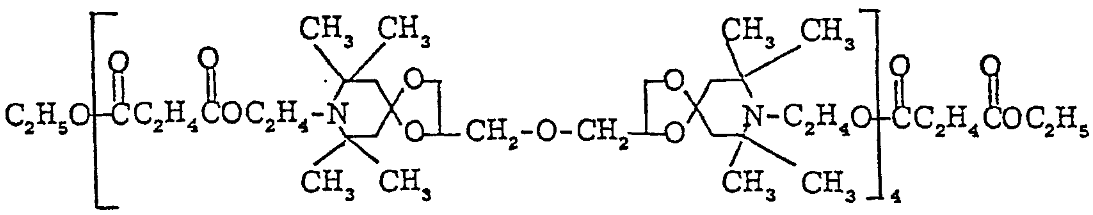 | 850 |
| 11 | 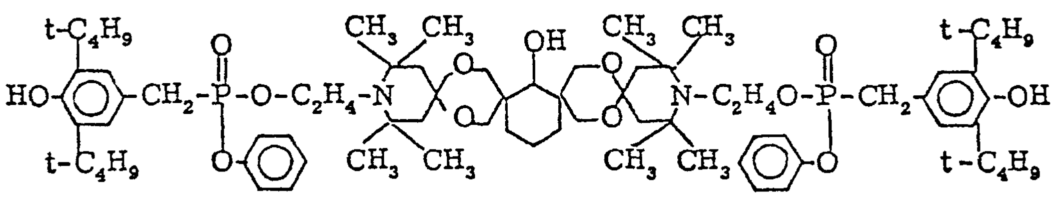 | 830 |
| 12 | 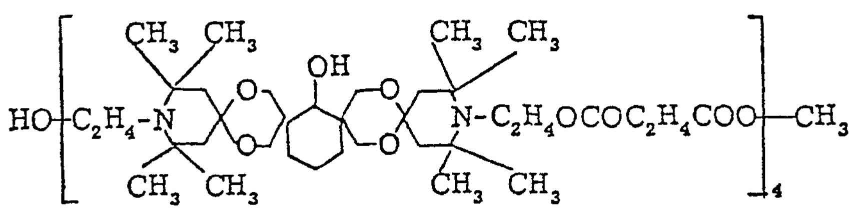 | 830 |

It is apparent that each of the stabilizers in accordance with the invention is far superior to the Controls.

Examples 13 to 26

Polypropylene compositions were prepared using stabilizers of the invention and of the prior art, and having the following formulation:

| Ingredient | Parts by Weight |
|---|---|
| Polypropylene | 100 |
| Stearyl β-3,5-di-tert-butyl-4-hydroxyphenyl priopionate | 0.2 |
| Stabilizer as shown in Table II | 0.3 |

The compositions were thoroughly blended in a Brabender® Plastograph, and then compression-molded to form sheets 0.3 mm thick. Pieces 2.5 $cm^2$ were cut off from the sheets and exposed to a high

pressure mercury lamp with and without immersion in hot water at 80°C for fifteen hours. The hours to failure were noted in comparison with four prior art stabilizers, and the results are shown in Table II.

TABLE II

| | Stabilizer | Hours to Failure Without Immersion | Hours to Failure After Immersion for 15 hours |
|---|---|---|---|
| Control 1 | Oxabis-(9-aza-3-hydroxymethyl-8,8,10,10-tetramethyl-1,5-dioxaspiro[5,5]-3-undecylmethane) | 620 | 520 |
| Control 2 | 9-Aza-3-ethyl-8,8,10,10-tetramethyl-1,5-dioxaspiro [5,5]-3-undecylmethyl-3-(3,5-di-t-butyl-4-hydroxyphenyl) propionate | 610 | 500 |
| Control 3 | Bis-(2,2,6,6-tetramethyl-4-piperidinyl) sebacate | 490 | 340 |
| Control 4 | Oxabis-(8-aza-7,7,9,9-tetramethyl-1,4-dioxaspiro [4,5]-2-decylmethane) | 605 | 515 |

TABLE II (continued)

| Example No. | Stabilizer | Hours to Failure: Without Immersion | Hours to Failure: After Immersion for 15 hours |
|---|---|---|---|
| 13 | 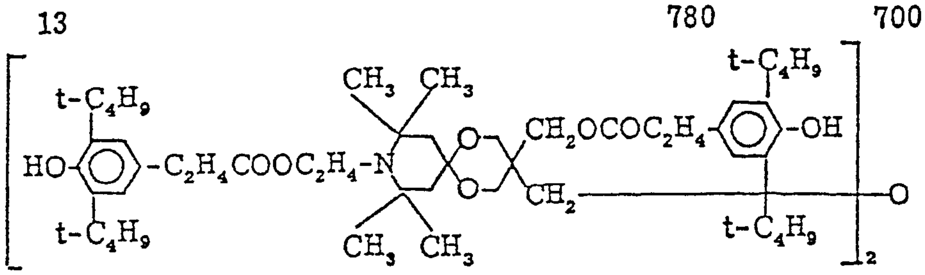 | 780 | 700 |
| 14 | 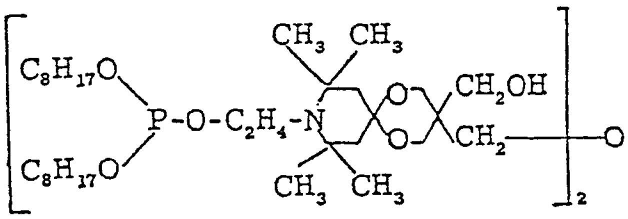 | 750 | 680 |
| 15 | 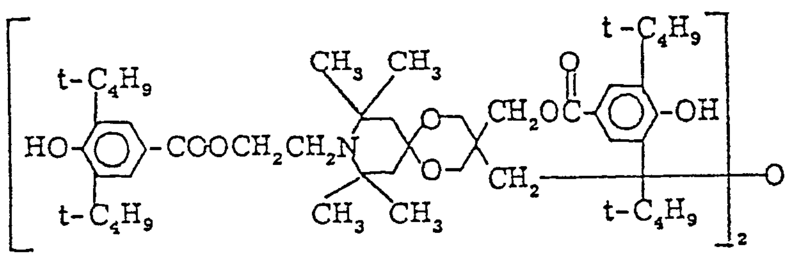 | 810 | 730 |
| 16 | 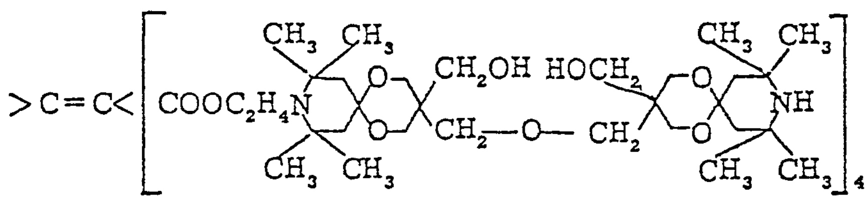 | 800 | 730 |
| 17 | 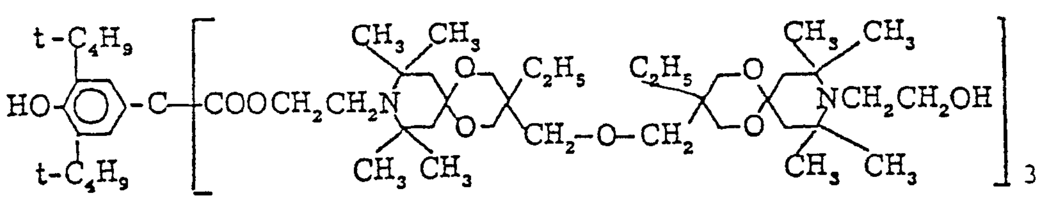 | 780 | 710 |

TABLE II (continued)

| Example No. | Stabilizer | Hours to Failure: Without Immersion | Hours to Failure: After Immersion for 15 hours |
|---|---|---|---|
| 18 | 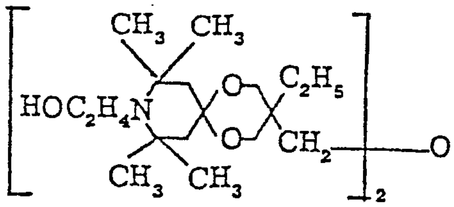 | 720 | 640 |
| 19 | 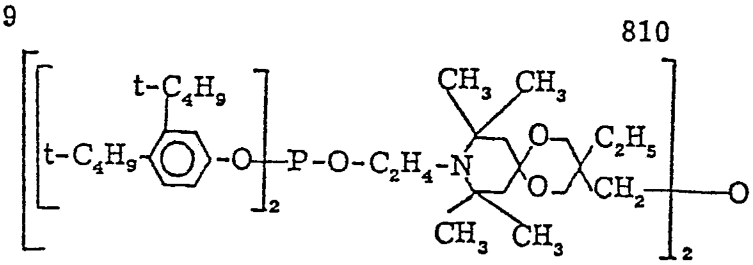 | 810 | 740 |
| 20 | 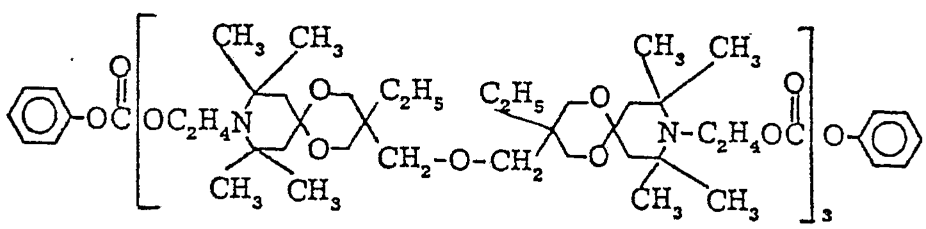 | 800 | 740 |
| 21 | 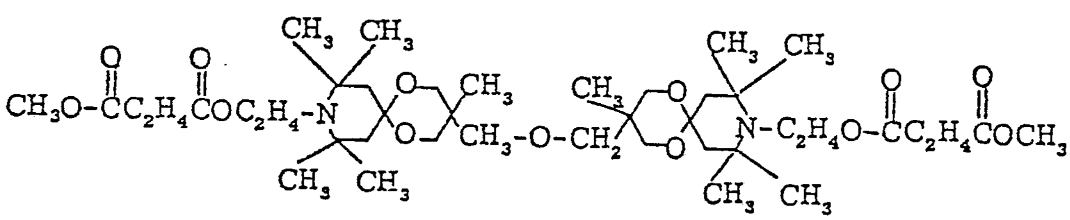 | 770 | 720 |
| 22 | 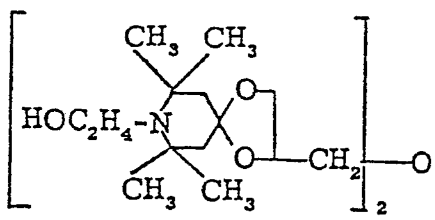 | 730 | 670 |

TABLE II (continued)

| Example No. | Stabilizer | Hours to Failure: Without Immersion | Hours to Failure: After Immersion for 15 hours |
|---|---|---|---|
| 23 | 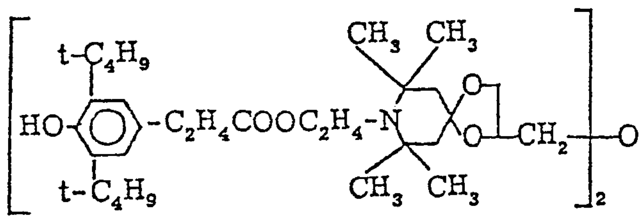 | 770 | 700 |
| 24 | 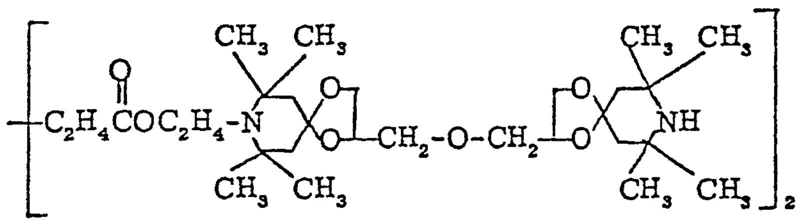 | 760 | 700 |
| 25 | 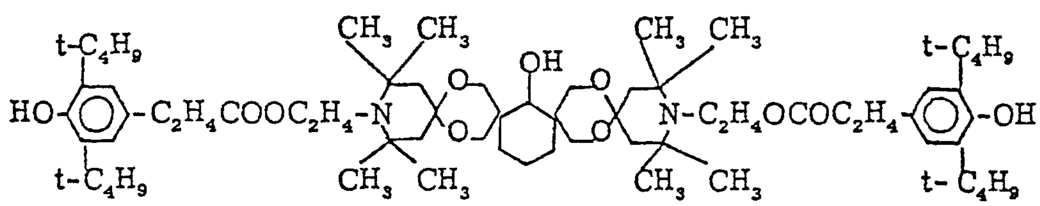 | 740 | 670 |
| 26 | 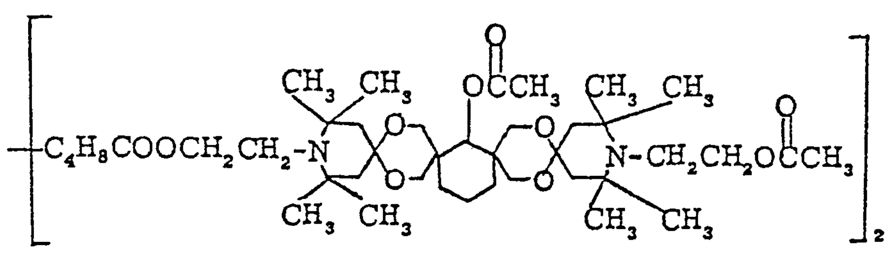 | 750 | 670 |

It is apparent that each of the stabilizers in accordance with the invention is far superior to the Controls.

Examples 27 to 40

Ethylene-vinyl acetate copolymer compositions were prepared using stabilizers of the invention and of the prior art, having the following formulation:

| Ingredient | Parts by Weight |
|---|---|
| Ethylene-vinyl acetate copolymer | 100 |
| 2,6-di-t-butyl-p-cresol | 0.1 |
| Ca stearate | 0.1 |
| Zn stearate | 0.1 |
| Diisodecylphenyl phosphite | 0.2 |
| Stabilizer as shown in Table III | 0.2 |

The stabilizer was blended with the polymer on a two-roll mill at 130°C, and sheets 0.4 mm thick were then compression molded at 140°C from the resulting blend. Pieces 2.5 $cm^2$ were cut off from the sheets and exposed to ultraviolet light in a Weather-O-Meter® for 500 hours. At the start and at the conclusion of the test, tensile strength of the sheet samples was determined.

The results in comparison with four controls, oxabis-(9-aza-3-hydroxymethyl-8,8,10,10-tetramethyl-1,5-dioxaspiro-[5,5]-3-undecylmethane), bis-(2,2,6,6-tetramethyl-4-piperidinyl) succinate, oxabis-(9-aza-3-ethyl-8,8,10,10-tetramethyl-1,5-dioxaspiro-[5,5]-3-undecylmethane) and condensed compound of 1-hydroxyethyl-2,2,6,6-tetramethyl-4-piperidinol with dimethyl succinate (molecular weight >2,000) are given in Table III as percent retention of the initially determined tensile strength:

## TABLE III

| | Stabilizer | % Retention of Tensile Strength After 500 Hours |
|---|---|---|
| Control 1 | Oxabis-(9-aza-3-hydroxy-methyl-8,8,10,10-tetramethyl-1,5-dioxaspiro-[5,5]-3-undecylmethane) | 70 |
| Control 2 | Bis-(2,2,6,6-tetramethyl-4-piperidinyl) succinate | 62 |
| Control 3 | Oxabis-(9-aza-3-ethyl-8,8,10,10-tetramethyl-1,5-dioxaspiro [5,5]-3-undecylmethane) | 68 |
| Control 4 | Condensed compound of 1-hydroxyethyl-2,2,6,6-tetramethyl-4-piperidinol with dimethyl succinate (Molecular weight ≻ 2,000) | 65 |
| Example No. | | |
| 27 | 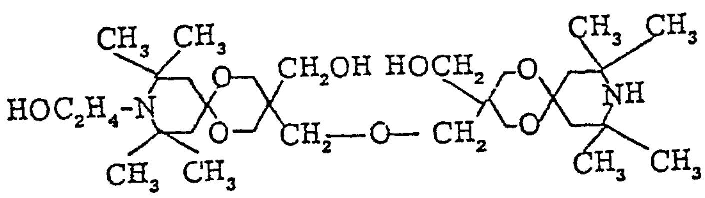 | 76 |
| 28 | 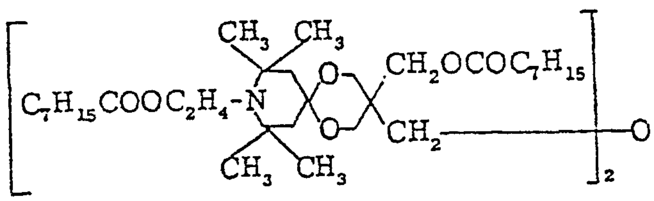 | 80 |
| 29 | 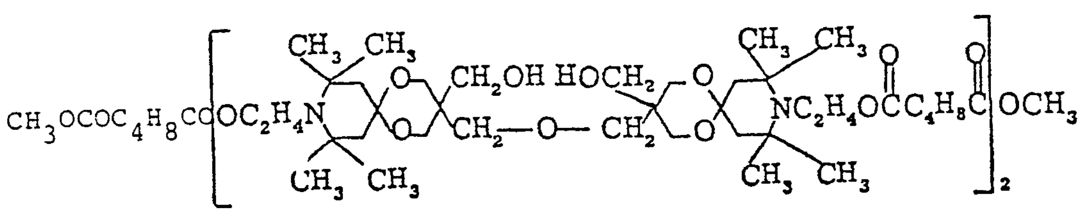 | 83 |

TABLE III (continued)

| Example No. | Stabilizer | % Retention of Tensile Strength After 500 Hours |
|---|---|---|
| 30 | 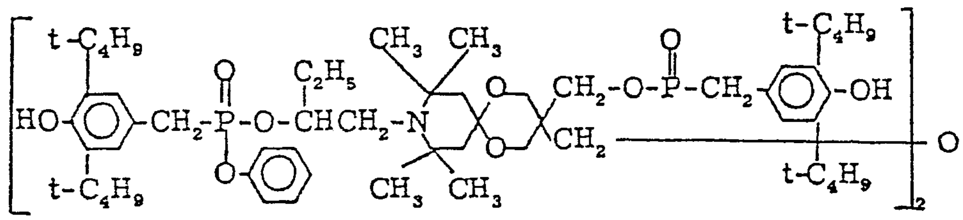 | 84 |
| 31 | 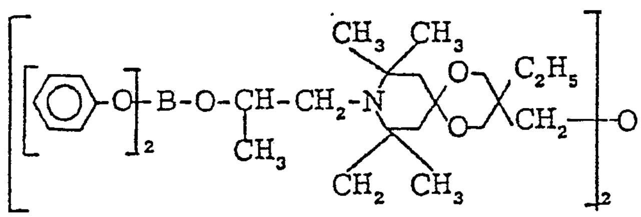 | 75 |
| 32 | 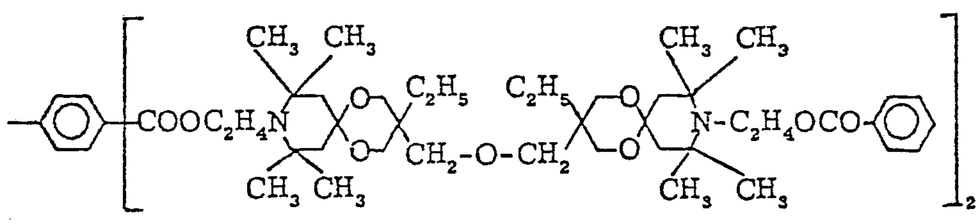 | 82 |
| 33 | 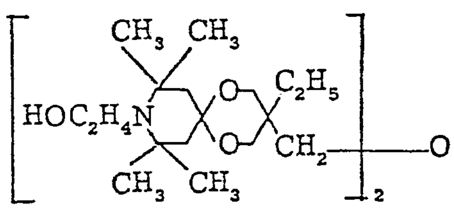 | 79 |
| 34 | 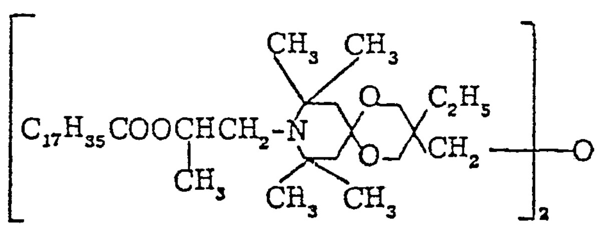 | 79 |

TABLE III (continued)

| Example No. | Stabilizer | % Retention of Tensile Strength After 500 Hours |
|---|---|---|
| 35 | 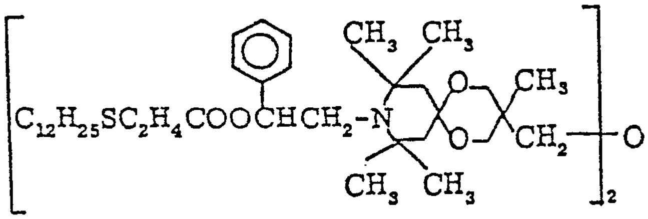 | 78 |
| 36 | 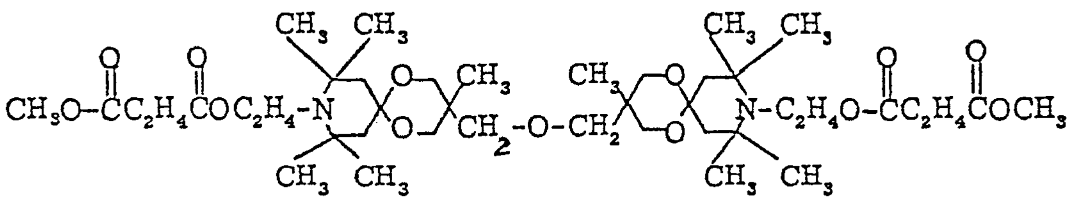 | 83 |
| 37 | 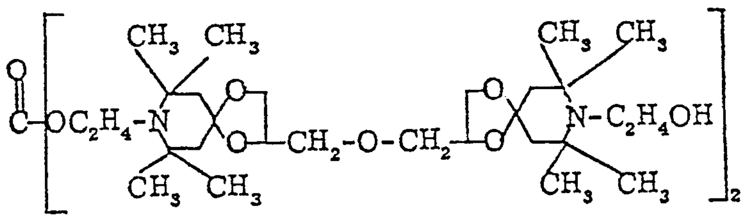 | 78 |
| 38 | 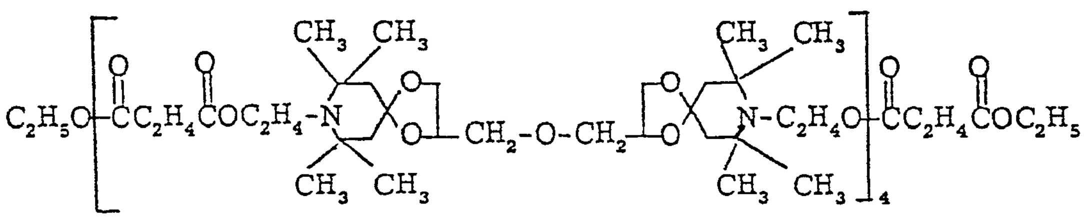 | 80 |
| 39 | 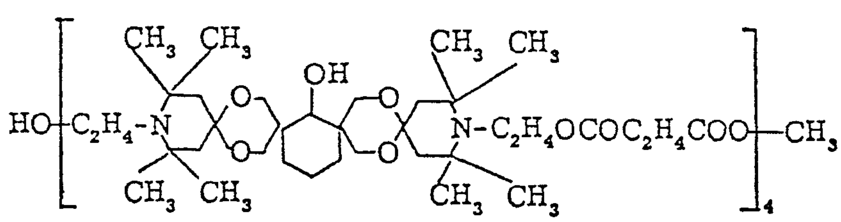 | 82 |
| 40 | 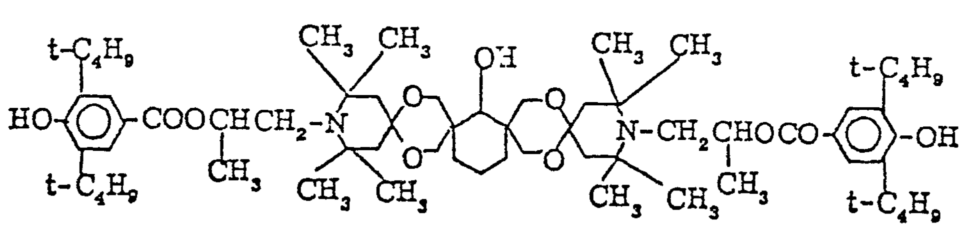 | 79 |

It is apparent that each of the stabilizers in accordance with the invention is far superior to the Controls.

Examples 41 to 53

High density polyethylene compositions were prepared using the stabilizers of the invention and of the prior art, and having the following formulation:

| Ingredient | Parts by Weight |
|---|---|
| High-density polyethylene | 100 |
| Ca stearate | 1 |
| Tetrakis-(methylene-3-(3,5-di-t-butyl-4-hydroxyphenyl)propionate)methane | 0.1 |
| Distearylthiodipropionate | 0.3 |
| Stabilizer as shown in Table IV | 0.2 |

The stabilizer was blended with the polymer on a two-roll mill and sheets 0.5 mm thick were prepared by compression-molding of the blend. Pieces 2.5 $cm^2$ were cut off from the sheets, and exposed in a Weather-O-Meter780 to ultraviolet light. The time in hours when degradation set in, as determined by a significant discolortion and/or embrittlement, was noted as hours to failure, and the results are reported in Table IV.

It is apparent that each of the stabilizers in accordance with the invention is far superior to the Controls.

TABLE IV

| | Stabilizer | Hours to Failure |
|---|---|---|
| Control 1 | Oxabis-(9-aza-3-ethyl-8,8,10,10-tetramethyl-1,5-dioxaspiro [5,5]-3-undecylmethane) | 870 |
| Control 2 | Tris-(9-aza-3-ethyl-8,8,10,10-tetramethyl-1,5-dioxaspiro [5,5]-3-undecylmethyl) phosphite | 900 |
| Control 3 | 2,2,4,4,18,18,20,20-Octamethyl-3,19-diaza-7,15,22,26-tetraoxa-24-hydroxy tetraspiro [5,2,3,2,5,2,1,2]-hexaeicosane | 860 |
| Control 4 | Bis-(2,2,6,6-tetramethyl-4-piperidinyl) adipate | 680 |
| Example No. | | |
| 41 | 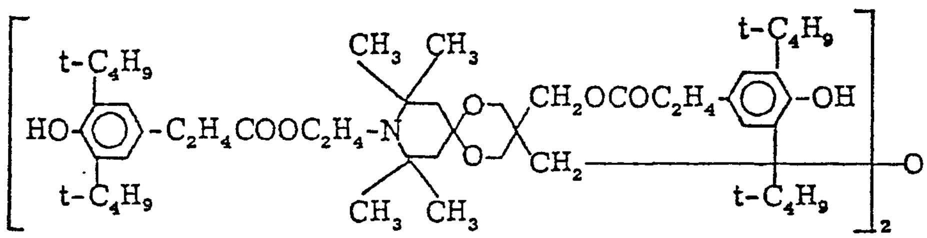 | 1150 |
| 42 | 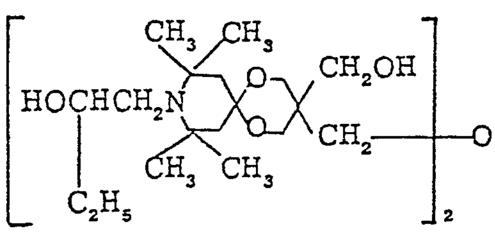 | 1050 |
| 43 | 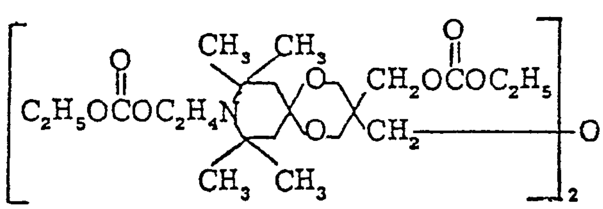 | 1060 |

TABLE IV (continued)

| Example No. | Stabilizer | Hours to Failure |
|---|---|---|
| 44 | 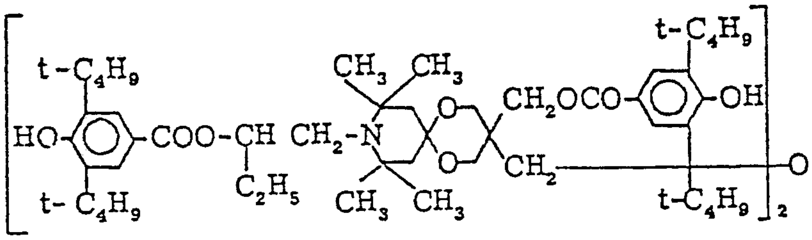 | 1090 |
| 45 | 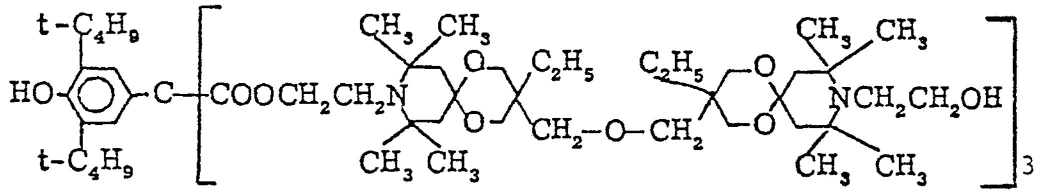 | 1170 |
| 46 | 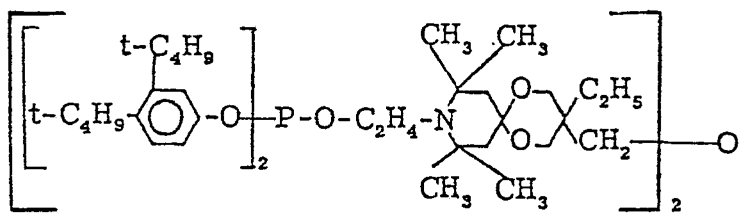 | 1200 |
| 47 | 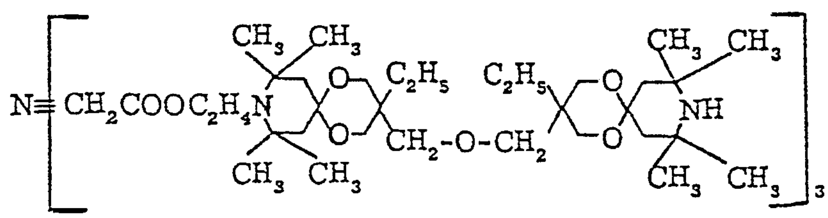 | 1140 |
| 48 | 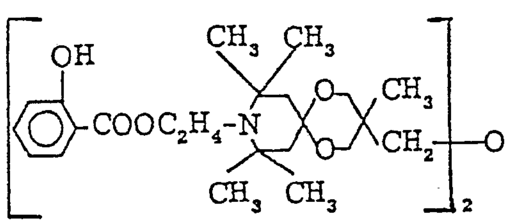 | 1100 |

TABLE IV (continued)

| Example No. | Stabilizer | Hours to Failure |
|---|---|---|
| 49 | 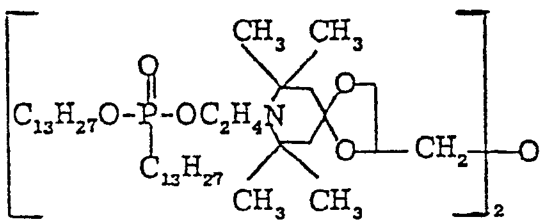 | 1070 |
| 50 | 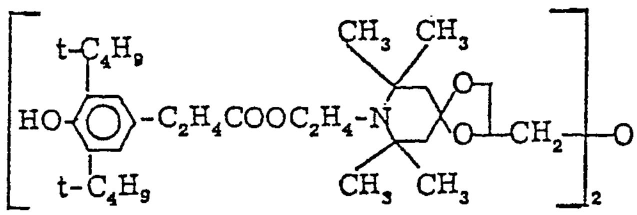 | 1150 |
| 51 | 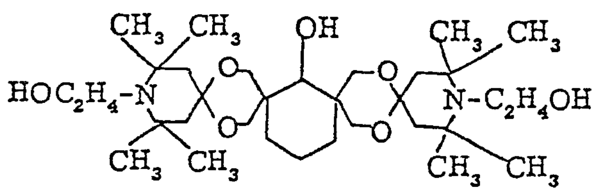 | 1040 |
| 52 | 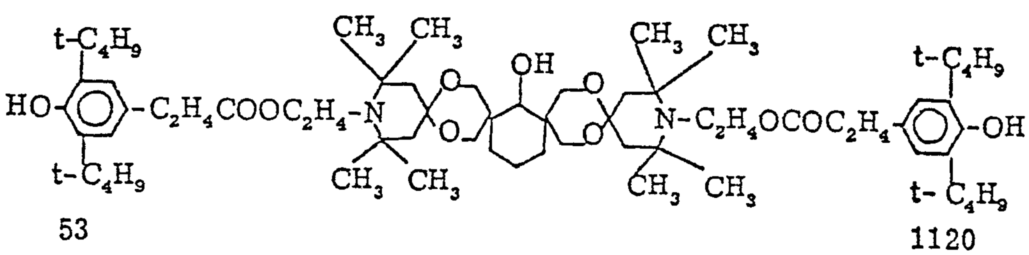 | 1180 |
| 53 | 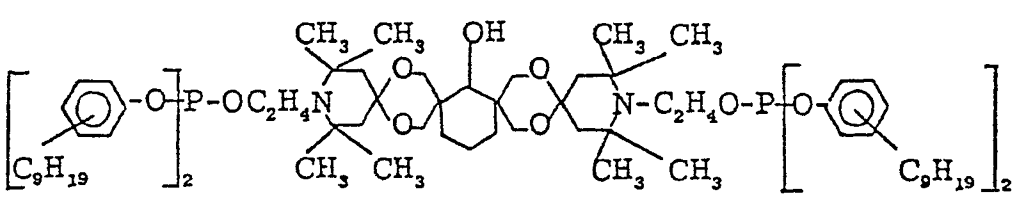 | 1120 |

Examples 54 to 66

Acrylonitrile-butadiene-styrene terpolymer resin compositions were prepared using stabilizers of the invention and of the prior art, and having the following formulation:

| Ingredient | Parts by Weight |
|---|---|
| Acrylonitrile-butadiene-styrene terpolymer | 100 |
| 4,4'-Butylidene-bis-(2-tert-butyl-m-cresol) | 0.1 |
| Stabilizer as shown in Table V | 0.3 |

The stabilizer was blended with the resin on a two-roll mill, and sheets 3 mm thick were prepared by compression-molding of the resulting blend. Pieces 2.5 $cm^2$ were cut off from the sheets, and subjected to ultraviolet light in a Weather-O-Meter® for 800 hours. Tensile strength before and after the test exposure was determined, and the results reported as the percent of tensile strength retained, at the end of this time, in Table V.

TABLE V

| | Stabilizer | % Tensile Strength Retained |
|---|---|---|
| Control 1 | Oxabis-(9-aza-3-hydroxy-methyl-8,8,10,10-tetramethyl-1,5-dioxaspiro [5,5]-3-undecyl-methane) | 65 |
| Control 2 | 2(2'-Hydroxy-5'-methylphenyl) benzotriazole | 63 |
| Control 3 | Bis-(9-aza-3-ethyl-8,8,10,10-tetramethyl-1,5-dioxaspiro [5,5]-3-undecylmethyl) carbonate | 62 |
| Control 4 | 3,15-Bis-(2-hydroxyethyl)2,2,4,4,14,14,16,16-octamethyl-3,15-diaza-7,11,18,21-tetraoxatrispiro-[5,2,2,5,2,2] heneicosane | 66 |

TABLE V (continued)

| Example No. | Stabilizer | % Tensile Strength Retained |
|---|---|---|
| 54 | 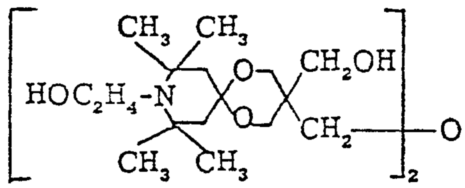 | 82 |
| 55 | 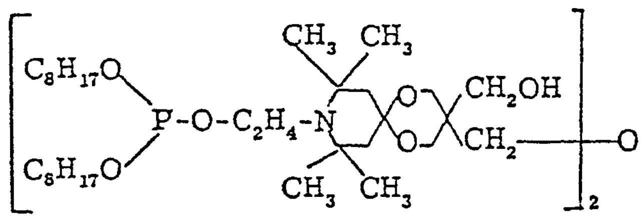 | 83 |
| 56 | 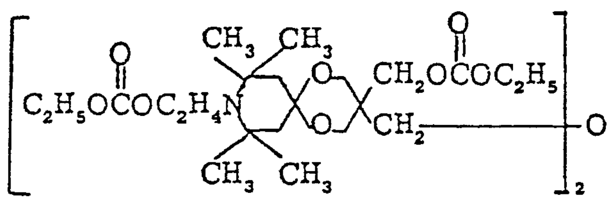 | 86 |
| 57 | 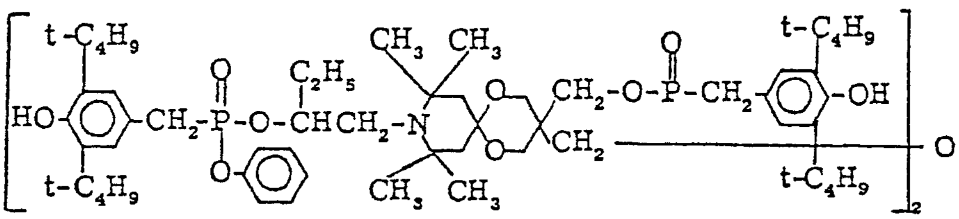 | 89 |
| 58 | 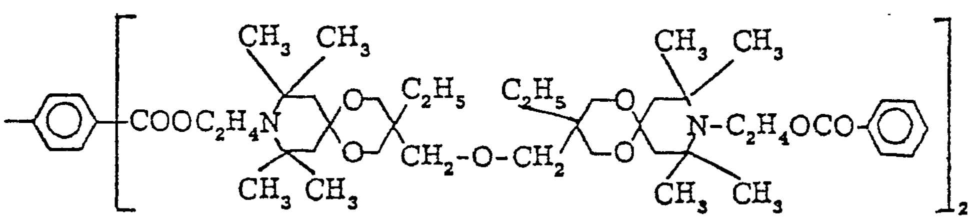 | 84 |
| 59 | 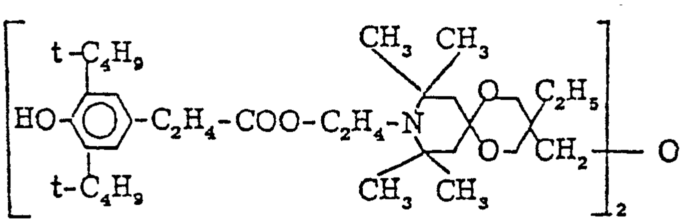 | 90 |

TABLE V (continued)

| Example No. | Stabilizer | % Tensile Strength Retained |
|---|---|---|
| 60 | 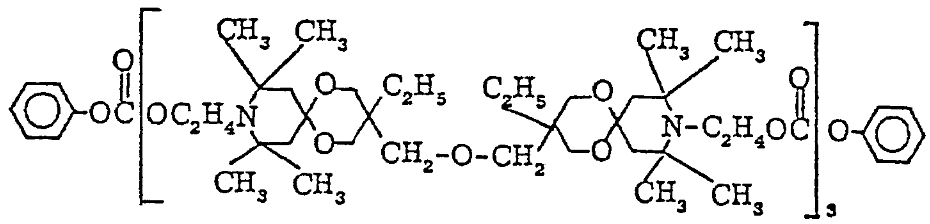 | 85 |
| 61 | 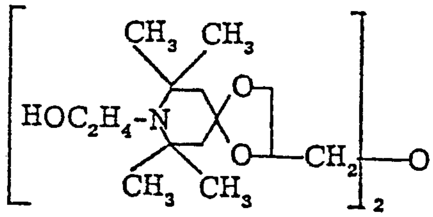 | 82 |
| 62 | 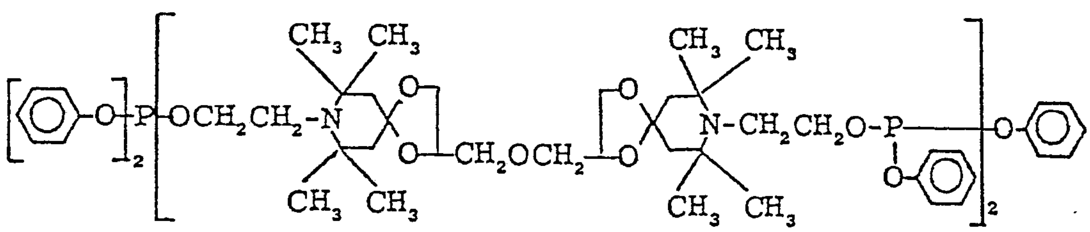 | 83 |
| 63 | 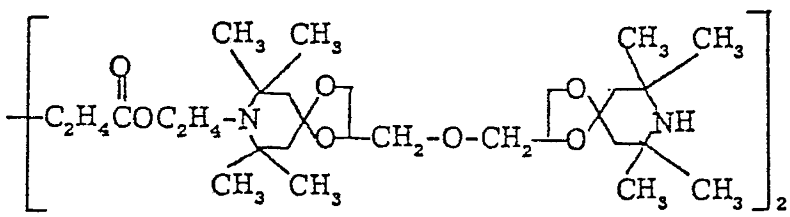 | 84 |
| 64 | 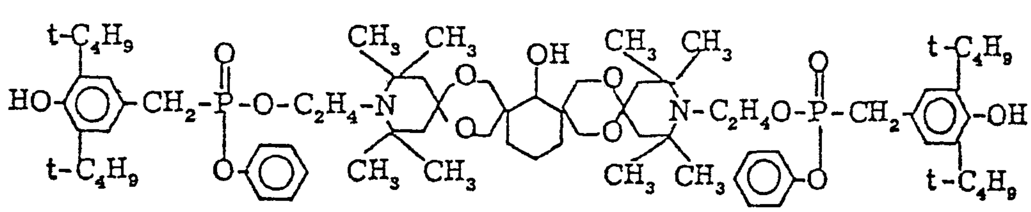 | 88 |

TABLE V (continued)

| Example No. | Stabilizer | % Tensile Strength Retained |
|---|---|---|
| 65 | 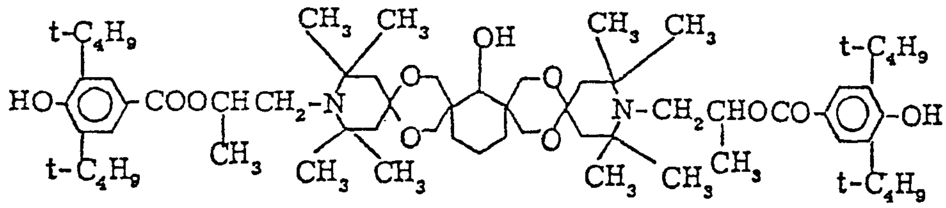 | 85 |
| 66 | 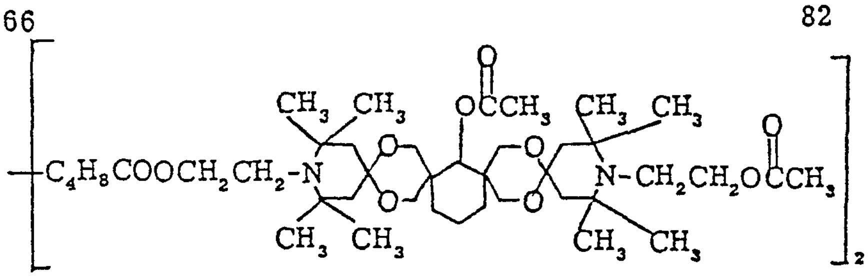 | 82 |

It is apparent that each of the stabilizers in accordance with the invention is far superior to the Controls.

Examples 67 to 77

Conventional heat stabilizers for polymeric materials may lose their effectiveness because of volatilization or decomposition at high polymer processing temperatures. This is not true of the stabilizers of the invention, as shown by observing the effect of heat in repeated extrusions of ethylene-propylene copolymer compositions. These compositions were prepared using stabilizers of the invention and of the prior art, and having the following formulation:

| Ingredient | Parts by Weight |
|---|---|
| Ethylene-propylene copolymer | 100 |
| Ca stearate | 0.2 |
| Stearyl-β-(3,5-di-t-butyl-4-hydroxyphenyl) propionate | 0.1 |
| Dilauryl thiodipropionate | 0.2 |
| Stabilizer as shown in Table VI | 0.2 |

The ingredients were mixed and the compositions then extruded (cylinder temperature 230°C and 240°C head die temperature 250°C, velocity 20 rpm) five times. Test pieces were then molded by injection molding at 250°C. The test pieces were exposed to a high voltage mercury lamp, and the hours to failure noted as shown in Table VI. The surface of the test pieces was also noted after exposure for 300 hours.

TABLE VI

| | Stabilizer | Hours to Failure Extruded 1 time | Extruded 5 times | Surface of test pieces after exposure for 500 hours |
|---|---|---|---|---|
| Control 1 | Bis-(9-aza-8,8,10,10-tetramethyl-3-ethyl-1,5-dioxaspiro [5,5]-3-undecylmethyl) adipate | 400 | 260 | Bloom |
| Control 2 | Bis-(9-aza-3-ethyl-8,8,10,10-tetramethyl-1,5-dioxaspiro [5,5]-3-undecylmethyl) carbonate | 380 | 250 | White spots |
| Control 3 | Oxabis-(9-aza-8,8,10,10-tetramethyl-3-hydroxymethyl-1,5-dioxaspiro [5,5]-3-undecylmethane) | 490 | 380 | White spots |
| Example No. | | | | |
| 67 | | 600 | 550 | No change |

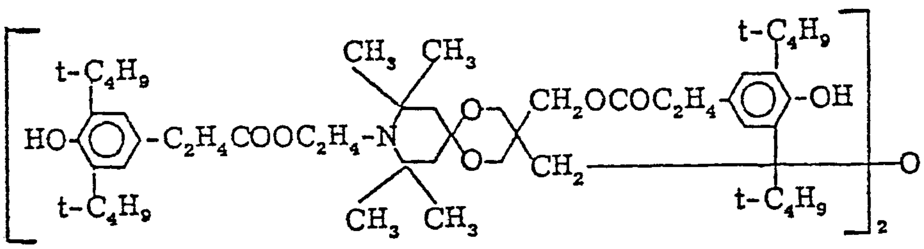

TABLE VI(continued)

| Example No. | Stabilizer | Hours to Failure: Extruded 1 time | Hours to Failure: Extruded 5 times | Surface of test pieces after exposure for 500 hours |
|---|---|---|---|---|
| 68 | 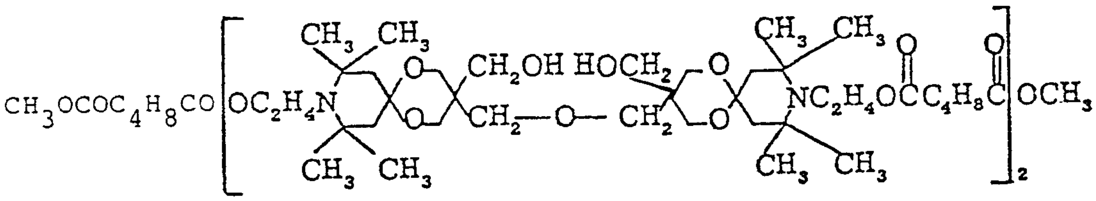 | 640 | 580 | No change |
| 69 | 11. 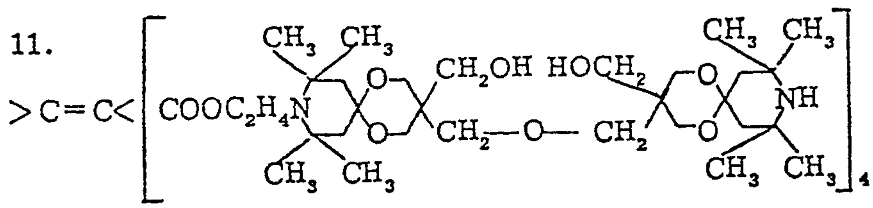 | 570 | 500 | No change |
| 70 | 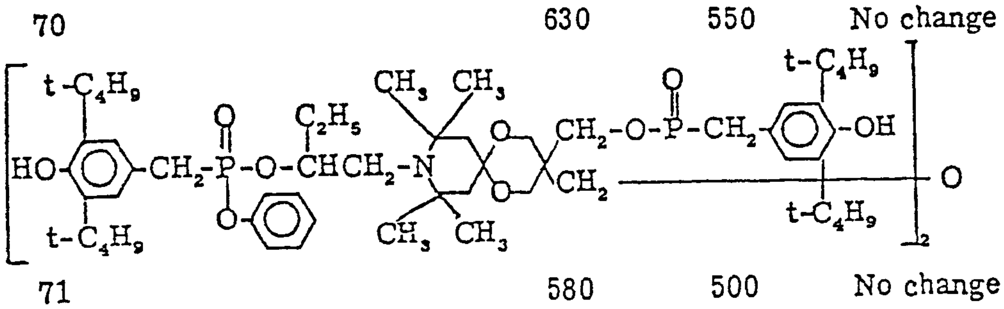 | 630 | 550 | No change |
| 71 | 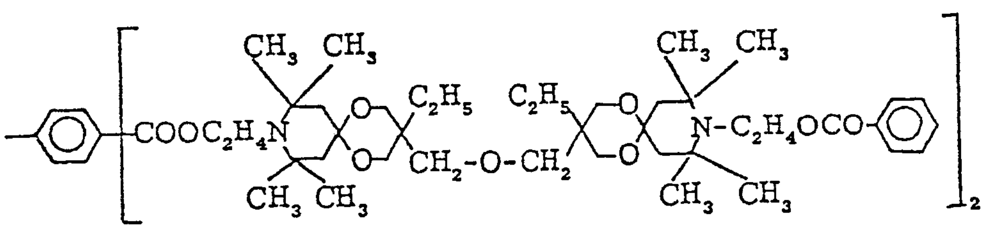 | 580 | 500 | No change |
| 72 | 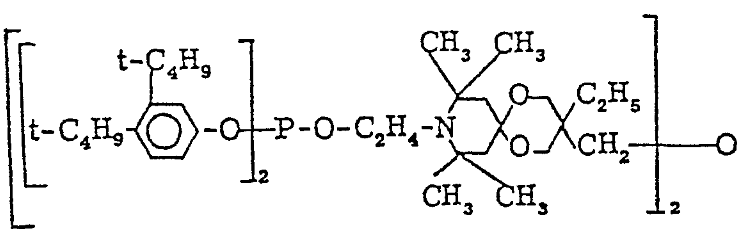 | 610 | 540 | No change |

TABLE VI (continued)

| Example No. | Stabilizer | Hours to Failure Extruded 1 time | Hours to Failure Extruded 5 times | Surface of test pieces after expos ure for 500 hours |
|---|---|---|---|---|
| 73 | 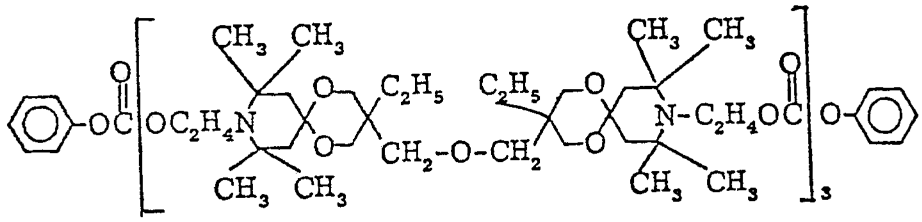 | 570 | 510 | No change |
| 74 | 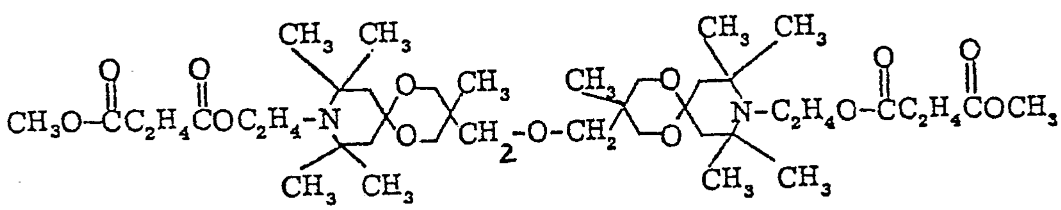 | 610 | 580 | No change |
| 75 | 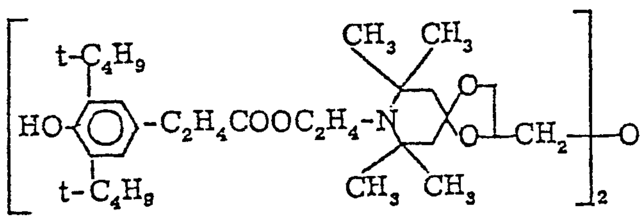 | 590 | 520 | No change |
| 76 | 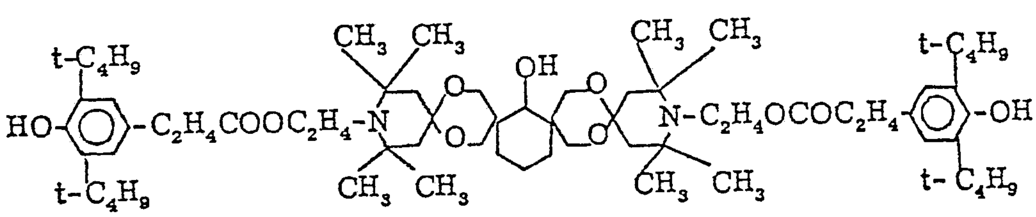 | 630 | 570 | No change |
| 77 | 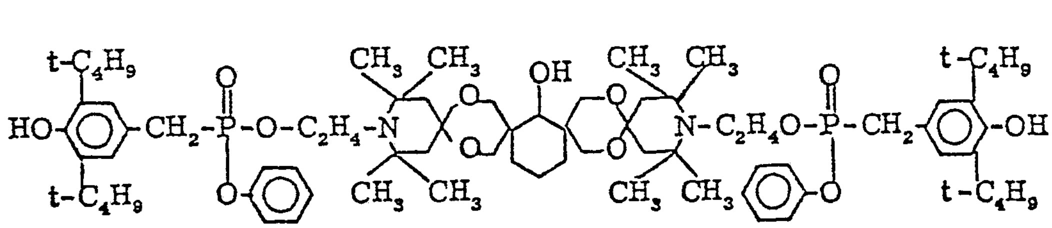 | 620 | 570 | No change |

The results show that substantial amounts of the Control stabilizers are lost by volatilization, after five extrusions, while the stabilizers of the invention are substantially retained in the polymer composition.

Examples 78 to 87

Polyurethane resin compositions were prepared using stabilizers of the invention and stabilizers of the prior art, and having the following formulation:

| Ingredient | Parts by Weight |
|---|---|
| Polyurethane resin (Asahi Denka U—100)[1] | 100 |
| Ca stearate | 0.7 |
| Sn stearate | 0.3 |
| 2,6-di-t-butyl-p-cresol | 0.1 |
| Stabilizer as shown in Table VII | 0.3 |

[1] A polyurethane-isocyanurate made from toluene diisocyanate and alkylene polyol.

The stabilizer was blended with the finely powdered polyurethane resin on a two-roll mill for five minutes at 70°C, and the sheet was then compression-molded at 120°C for five minutes to form sheets 0.5 mm thick. Pieces 2.5 $cm^2$ were cut out from the sheets, and exposed to ultraviolet light in a Weather-O-Meter® for thirty hours. Elongation before and after exposure was determined, and the percent elongation retained after the exposure is given in Table VII.

TABLE VII

| | Stabilizer | % Elongation Retention |
|---|---|---|
| Control 1 | Bis-(9-aza-3-ethyl-8,8,10,10-tetramethyl-1,5-dioxaspiro [5,5]-3-undecylmethyl) adipate | 58 |
| Control 2 | Bis-(2,2,6,6-tetramethyl-4-piperidinyl)-1,6-hexamethylene dicarbamate | 55 |
| Control 3 | Oxabis-(9-aza-3-hydroxymethyl-8,8,10,10-tetramethyl-1,5-dioxaspiro [5,5]-3-undecylmethane) | 64 |
| Control 4 | 9-Aza-9-(3,5-di-t-butyl-4-hydroxyphenylpropionyloxy) ethyl-3-ethyl-8,8,10,10-tetramethyl-1,5-dioxaspiro [5,5]-3-undecylmethyl-3,5-di-t-butyl-4-hydroxyphenylpropionate | 60 |
| Example No. | | |
| 78 | 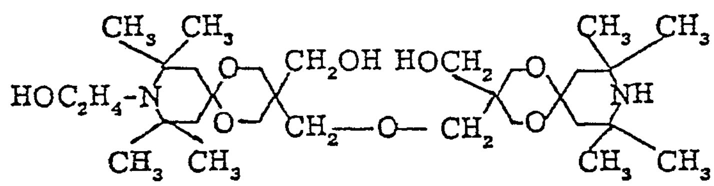 | 74 |
| 79 | 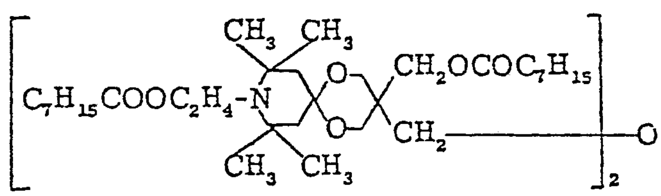 | 77 |
| 80 | 8. 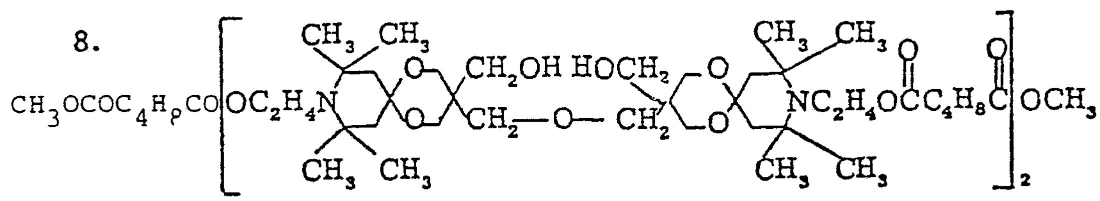 | 79 |

TABLE VII (continued)

| Example No. | Stabilizer | % Elongation Retention |
|---|---|---|
| 81 | 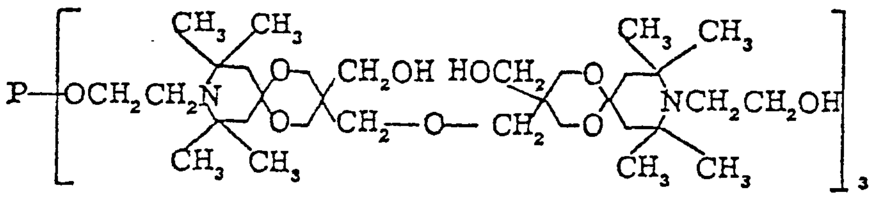 | 76 |
| 82 | 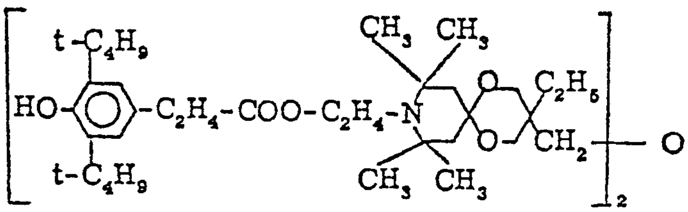 | 80 |
| 83 | 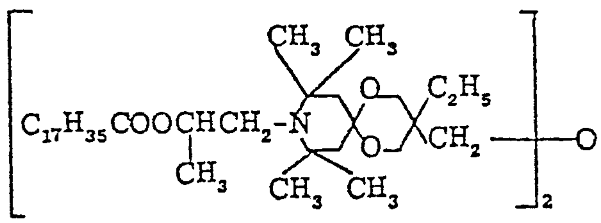 | 77 |
| 84 | 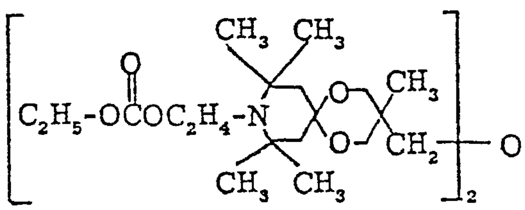 | 74 |
| 85 | 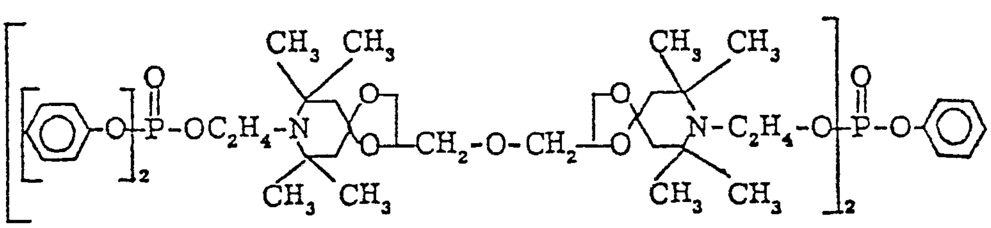 | 73 |

TABLE VII (continued)

| Example No. | Stabilizer | % Elongation Retention |
|---|---|---|
| 86 | 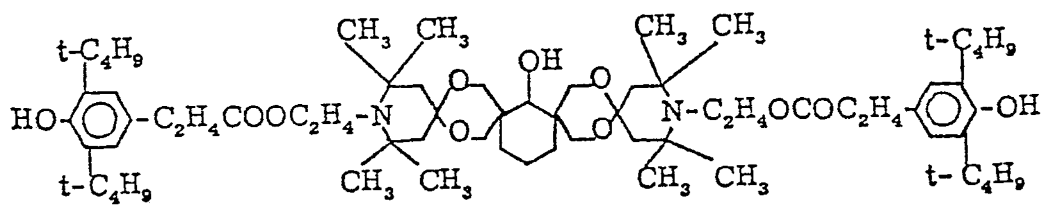 | 76 |
| 87 | 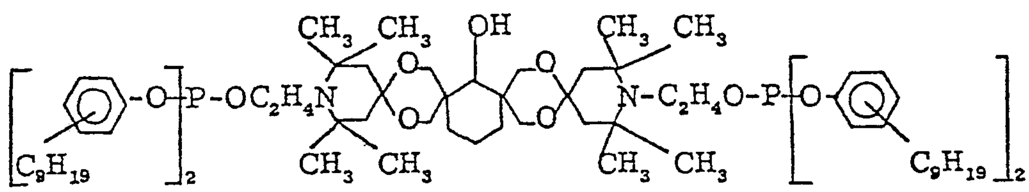 | 78 |

The stabilizers of the invention are clearly superior to the Controls in enhancing resistance of the polyurethane resin to degradation under ultraviolet light.

## Claims

1. Spiro[2,2,6,6-tetramethyl piperidine-4,2'-(1',3'-diox(ol)an] derivatives having the general formula:

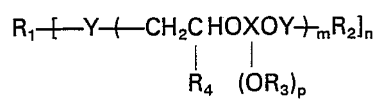 (I)

wherein:

Y is

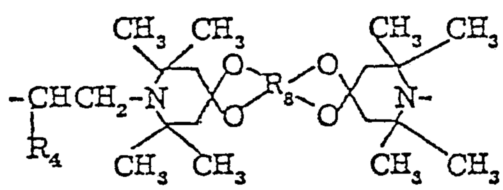

X is selected from the group consisting of alkylene groups having from 1 to 18 carbon atoms; cycloalkylene groups having from 3 to 8 carbon atoms, arylene or aralkylene groups having from 6 to 18 carbon atoms; polyacyl groups

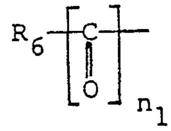

wherein $n_1$ is 2, 3 or 4; polycarbamoyl groups

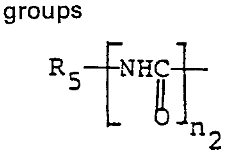

wherein $n_2$ is 2, 3 or 4,

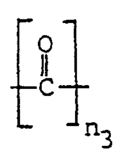

wherein $n_3$ is 1 or 2;

$$-\overset{O}{\overset{\|}{C}}OR_{11}O\overset{O}{\overset{\|}{C}}-;$$

the residues of the polyhydric compounds diethylene glycol, triethylene glycol, thiodiethylene glycol, 1,4-cyclohexanedimethanol, hydrogenated Bisphenol-A and tris(2-hydroxyethyl)isocyanurate; and di- and trivalent oxyacid groups derived from phosphorus-containing oxyacids, silicic acid or boric acid and esters thereof as well as dimers thereof joined by polyhydric alcohols and phenols;

$R_1$ is selected from the group consisting of hydroxy,

$$R_7O\overset{O}{\overset{\|}{C}}-O-,\quad (R_7O\overset{O}{\overset{\|}{C}}\,)_{q}R_{12}(\overset{O}{\overset{\|}{C}}-O)_{n},\quad (R_7O)_{2-n}\underset{R_{13}}{\underset{|}{\overset{(O)_r}{\overset{\|}{P}}}}(O)_{n} \text{ and } (R_7O)_{3-n}-B(O)_{n};$$

$R_2$ is selected from the group consisting of hydrogen and

$$-CH_2\underset{R_4}{\underset{|}{C}}HOR_{10};$$

$R_3$ is selected from the group consisting of alkyl groups having from 1 to 18 carbon atoms; cycloalkyl groups having from 3 to 8 carbon atoms; aryl or aralkyl groups having from 6 to 18 carbon atoms; and $-X-O-R_1$;

$R_4$ is selected from the group consisting of hydrogen; aliphatic groups having from 1 to 18 carbon atoms; cycloaliphatic groups having from 3 to 8 carbon atoms; and aromatic or araliphatic groups having from 6 to 18 carbon atoms;

$R_5$ is selected from the group consisting of aliphatic groups having from 1 to 18 carbon atoms; cycloaliphatic groups having from 3 to 8 carbon atoms, and aromatic or araliphatic groups having from 6 to 18 carbon atoms; and the residues of lysinediisocyanate, 3-isocyanatomethyl-3,5,5-trimethylcyclohexylisocyanate, bis-(isocyanatomethyl)-cyclohexane, 3-(2'-isocyanatocyclohexyl)propylisocyanate and diphenyletherdiisocyanate;

$R_6$ is selected from the group consisting of the residues of saturated or unsaturated aliphatic di-, tri- or tetracarboxylic acids, said residues having up to 18 carbon atoms; saturated or unsaturated cycloaliphatic di-, tri- or tetracarboxylic acids, said residues having up to 8 carbon atoms; aromatic or araliphatic di-, tri- or tetracarboxylic acids, said residues having up to 18 carbon atoms; and the residues of iminodiacetic acid, thiodipropionic acid, diglycolic acid, thiophene dicarboxylic acid, furane dicarboxylic acid, dicarboxyethylpiperidine and nitrilotriacetic acid;

$R_7$ is selected from the group consisting of the residues of monohydric alcohols having from 1 to 18 carbon atoms and optionally (cyclo)alkyl or halogen substituted phenols having altogether from 6 to 50 carbon atoms;

$R_8$ is selected from the group consisting of

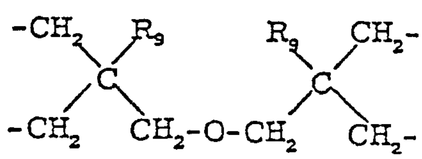

,

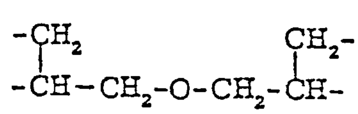

and

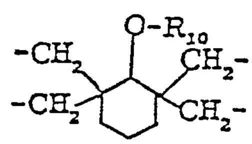

$R_9$ is selected from the group consisting of aliphatic groups having from 1 to 18 carbon atoms, cycloaliphatic groups having from 3 to 8 carbon atoms; and $-CH_2OR_{10}$;

$R_{10}$ is selected from the group consisting of hydrogen,

$$-\overset{O}{\overset{\|}{C}}-R_{12}(\overset{O}{\overset{\|}{C}}OR_7)_{n+q-1},\quad -\overset{O}{\overset{\|}{C}}OR_7,\quad -\underset{R_{13}}{\underset{|}{\overset{(O)_r}{\overset{\|}{P}}}}-OR_7, \text{ and } -B(OR_7)_2;$$

$R_{11}$ is selected from the group consisting of alkylene and oxyalkylene groups having from 2 to 10 carbon atoms and from 0 to 5 oxyether groups; cycloalkylene groups having from 3 to 8 carbon atoms; and optionally hydrocarbyl substituted arylene groups having altogether from 6 to 50 carbon atoms, including optionally hydrocarbyl substituted phenylene groups joined by an oxygen or sulfur atom or an optionally hydrocarbyl substituted methylene group; isocyanurate; and the residues of thiodiethylene glycol, 1,4-phenyldimethanol, hydrogenated Bisphenol-A, bis - (2 - methyl - 4 - hydroxy - 5 - t - butylbenzyl) - sulfide, 1,1,3 - tris - (2′ - methyl - 4′ - hydroxy - 5′ - t - butylphenyl) butane, 2,2 - bis(3′ - t - butyl - 4′ - hydroxyphenyl) - 4 - (3″,5″ - di - t - butyl - 4″ - hydroxyphenyl)butane and 2,2 - bis - (2′ - methyl - 5′ - t - butyl - 4′ - hydroxyphenyl) - 4 - (3″,5″ - di - t - butyl - 4″ - hydroxyphenyl)butane;

$R_{12}$ is the residue of a saturated or unsaturated (cyclo)aliphatic, aromatic or N-, O- or S-heteroaromatic mono-, di- tri- or tetracarboxylic acid, having from 2 to 22 carbon atoms and optionally containing keto or hydroxy groups; or the residue of aminoacetic acid, dodecyl mercaptopropionic acid, 3,5 - di - t - butyl - 4 - hydroxyphenyl - propionic acid, 2,2,6,6 - tetramethyl - piperidine - 4 - carboxylic acid, 3,8,8,10,10 - pentamethyl - 9 - aza - 1,5 - dioxaspiro[5,5]undecane - 3 - carboxylic acid, iminodiacetic acid, thiodipropionic acid, diglycolic acid, dicarboxyethyl piperidine and nitrilotriacetic acid;

$R_{13}$ is selected from the group consisting of hydrogen; alkyl or alkoxy groups having from 1 to 18 carbon atoms; cycloalkyl or cycloalkoxy groups having from 3 to 8 carbon atoms; aromatic, O-aromatic or araliphatic and O-araliphatic groups having from 6 to 18 carbon atoms;

$$R_7{-}O{-}\underset{\underset{O}{\|}}{C}{-}(O){-};$$

monovalent oxyacid groups derived from phosphorus containing acids, silicic acid or boric acid; and (4-hydroxy-3,5-di-tert.-butyl phenyl)-methyl;

m is a number from zero to 10;

n is a number from 1 to 4;

p is zero, 1 or 2;

q is a number from zero to 3; and

r is zero or 1.

2. Compounds according to claim 1 wherein m is zero or 1.

3. Compounds according to any of claims 1—2 in which $R_4$ is hydrogen or $R_4$ and/or $R_6$ are an aliphatic group having from 1 to 18 carbon atoms.

4. Compounds according to any of claims 1—3 wherein $R_1$ is hydroxy and/or $R_2$ is hydrogen.

5. Compounds according to any of claims 1—4, wherein

$R_1$ is

$$R_7{-}O{-}\underset{\underset{O}{\|}}{C}{-}O{-}\ ;\ (R_7{-}O{-}\underset{\underset{O}{\|}}{C})_qR_{12}(\underset{\underset{O}{\|}}{C}{-}O)_n{-}\ \ (R_7{-}O)_{2-n}{-}\overset{\overset{(O)_n}{\|}}{\underset{\underset{R_{13}}{|}}{P}}{-}(O)_n{-}\ \text{and } R_2 \text{ is } {-}CH_2{-}\underset{\underset{R_4}{|}}{CH}{-}OR_{10}.$$

6. Compound according to claim 1 having the structure:

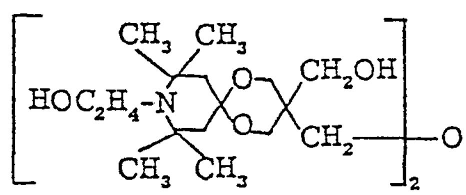

7. A polyvinyl chloride resin composition having improved resistance to deterioration, especially when heated at 177°C (250°F), comprising a polyvinyl chloride resin formed at least in part of the recurring group:

$$-\underset{\underset{Cl}{|}}{CH}-\overset{\overset{X}{|}}{\underset{\underset{X}{|}}{C}}-$$

and having a chlorine content in excess of 40%, wherein X is either hydrogen or chlorine, and a compound in accordance with any of the claims 1—6.

8. An olefin polymer composition having improved resistance to deterioration comprising an olefin polymer selected from the group consisting of polymers of alpha-olefins having from 2 to 6 carbon atoms, and a compound in accordance with any of the claims 1—6.

9. An acrylonitrile-butadiene-styrene terpolymer composition having improved resistance to deterioration comprising acrylonitrile-butadiene-styrene terpolymer and a compound in accordance with any of the claims 1—6.

10. An ethylene-vinyl acetate copolymer composition having improved resistance to deterioration comprising ethylene vinyl acetate copolymer and a compound in accordance with any of the claims 1—6.

11. A polyurethane resin composition having improved resistance to deterioration comprising a polyurethane resin and a compound in accordance with any of the claims 1—6.

**Patentansprüche**

1. Spiro[2,2,6,6-tetramethyl-piperidin-4,2'-(1',3'-diox(ol)an] derivate mit der allgemeinen Formel

$$R_1\!-\!\!\left[\!-Y\!-\!\left(CH_2\underset{\displaystyle R_4}{\underset{|}{C}}HO\underset{\displaystyle (OR_3)_p}{\underset{|}{X}}OY\right)_{\!m}\!R_2\right]_n \qquad (I)$$

worin

Y

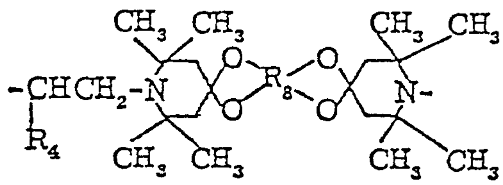

ist;

X ausgewählt ist aus der Gruppe aus Alkylengruppen mit 1 bis 18 Kohlenstoffatomen; Cycloalkylengruppen mit 3 bis 8 Kohlenstoffatomen; Arylen- oder Aralkylen-gruppen mit 6 bis 18 Kohlenstoffatomen; Polyacylgruppen

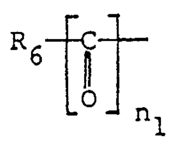

worin $N_1 = 2$, 3 oder 4 ist; Polycarbamoylgruppen

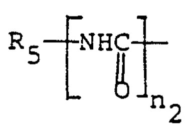

worin $N_2 = 2$, 3 oder 4 ist;

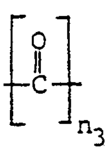

worin $n_3 = 1$ oder 2 ist;

$$-\overset{\displaystyle O}{\overset{\|}{C}}OR_{11}O\overset{\displaystyle O}{\overset{\|}{C}}-;$$

den Resten der mehrfunktionellen Verbindungen Diethylenglykol, Triethylenglykol, Thiodiethylenglykol, 1,4-Cyclohexandimethanol, hydriertem Bisphenol-A und Tris-(2-hydroxyethyl)isocyanurat und zwei- und dreiwertigen Oxysäuregruppen, abgeleitet von phosphorhaltigen Oxysäuren, Kieselsäure oder Borsäure, und Estern derselben sowie Dimeren derselben, die durch mehrwertige Alkohole und Phenole verbunden sind,

$R_1$ ausgewählt ist aus der aus Hydroxy,

$$R_7O\overset{\displaystyle O}{\overset{\|}{C}}-O-,\ \left(R_7O\overset{\displaystyle O}{\overset{\|}{C}}\right)_{\!q}R_{12}\!\left(\overset{\displaystyle O}{\overset{\|}{C}}-O\right)_{\!n},\ (R_7O)_{2-n}\!-\!\overset{\displaystyle (O)_r}{\overset{\|}{\underset{\displaystyle R_{13}}{\underset{|}{P}}}}\!-\!(O)_n \text{ und } (R_7O)_{3-n}\!-\!B\!-\!(O)_n\ ;$$

bestehenden Gruppe;

$R_2$ ausgewählt ist aus der aus Wasserstoff und

$$-CH_2\underset{\begin{array}{c}|\\R_4\end{array}}{CH}OR_{10}$$

bestehenden Gruppe;

$R_3$ ausgewählt ist aus der aus Alkylgruppen mit 1 bis 18 Kohlenstoffatomen; Cycloalkylgruppen mit 3 bis 8 Kohlenstoffatomen; Aryl- oder Aralkylgruppen mit 6 bis 18 Kohlenstoffatomen; und $-X-O-R_1$ bestehenden Gruppe;

$R_4$ ausgewählt ist aus der aus Wasserstoff; aliphatischen Gruppen mit 1 bis 18 Kohlenstoffatomen; cycloaliphatischen Gruppen mit 3 bis 8 Kohlenstoffatomen; und aromatischen oder araliphatischen Gruppen mit 6 bis 18 Kohlenstoffatomen bestehenden Gruppe;

$R_5$ ausgewählt ist aus der aus aliphatischen Gruppen mit 1 bis 18 Kohlenstoffatomen; cycloaliphatischen Gruppen mit 3 bis 8 Kohlenstoffatomen; und aromatischen oder araliphatischen Gruppen mit 6 bis 18 Kohlenstoffatomen; sowie den Resten von Lysindiisocyanat, 3-Isocyanatomethyl-3,5,5-trimethyl-cyclohexylisocyanat, Bis-(isocyanatomethyl)-cyclohexan, 3-(2'-Isocyanatocyclohexyl)-propylisocyanat und Diphenyletherdiisocyanat bestehenden Gruppe;

$R_6$ ausgewählt ist aus der Gruppe aus den Resten gesättigter oder ungesättigter aliphatischer Di-, Tri- oder Tetracarbonsäuren, wobei diese Reste bis zu 18 Kohlenstoffatome aufweisen; gesättigter oder ungesättigter cycloaliphatischer Di-, Tri- oder Tetracarbonsäuren, wobei diese Reste bis zu 8 Kohlenstoffatome aufweisen; aromatischer oder araliphatischer Di-, Tri- oder Tetracarbonsäuren, wobei diese Reste bis zu 18 Kohlenstoffatome aufweisen; und den Resten von Iminodiessigsäure, Thiodipropionsäure, Diglykolsäure, Thiophendicarbonsäure, Furandicarbonsäure, Dicarboxyethylpiperidin und Nitrilotriessigsäure;

$R_7$ ausgewählt ist aus der Gruppe aus den Resten einwertiger Alkohole mit 1 bis 18 Kohlenstoffatomen und gegebenenfalls (cyclo)-alkyl- oder halogensubstituierten Phenolen mit insgesamt 6 bis 50 Kohlenstoffatomen;

$R_8$ ausgewählt ist aus der aus

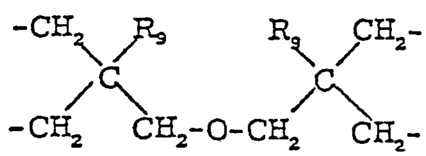 , 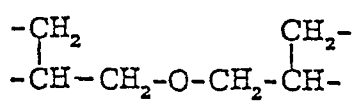 und

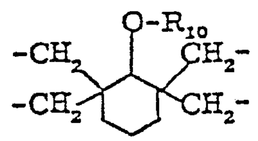

bestehenden Gruppe;

$R_9$ ausgewählt ist aus der aus aliphatischen Gruppen mit 1 bis 18 Kohlenstoffatomen, cycloaliphatischen Gruppen mit 3 bis 8 Kohlenstoffatomen und $-CH_2OR_{10}$ bestehenden Gruppe;

$R_{10}$ ausgewählt ist aus der aus Wasserstoff,

$$-\overset{\overset{O}{\|}}{C}-R_{12}\left(\overset{\overset{O}{\|}}{C}OR_7\right)_{n+q-r},\quad -\overset{\overset{O}{\|}}{C}OR_7,\quad -\underset{\underset{R_{13}}{|}}{\overset{\overset{(O)_r}{\|}}{P}}-OR_7, \text{ und } -B(OR_7)_2;$$

bestehenden Gruppe:

$R_{11}$ ausgewählt ist aus der Gruppe aus Alkylen- und Oxyalkylengruppen mit 2 bis 10 Kohlenstoffatomen und 0 bis 5 Oxyethergruppen; Cycloalkylengruppen mit 3 bis 8 Kohlenstoffatomen; und gegebenenfalls hydrocarbylsubstituierten Arylengruppen mit insgesamt 8 bis 50 Kohlenstoffatomen, einschließlich gegebenenfalls hydrocarbylsubstituierten Phenylengruppen, die durch ein Sauerstoff- oder Schwefelatom oder eine gegebenenfalls hydrocarbylsubstituierte Methylengruppe verbunden sind; Isocyanurat; und den Resten von Thiodiethylenglykol, 1,4 - Phenyldimethanol, hydriertem Bisphenol-A, bis - (2 - methyl - 4 - hydroxy - 5 - t - butylbenzyl)sulfid, 1,1,3 - Tris - (2' - methyl - 4' - hydroxy - 5' - t - butylphenyl) - butan, 2,2 - Bis - (3' - t - butyl - 4' - hydroxyphenyl) - 4 - (3'',5'' - di - t - butyl - 4'' - hydroxyphenyl) - butan und 2,2 - Bis - (2' - methyl - 5' - t - butyl - 4' - hydroxyphenyl) - 4 - (3'',5'' - di - t - butyl - 4'' - hydroxyphenyl) - butan;

$R_{12}$ der Rest einer gesättigten oder ungesättigten (cyclo)aliphatischen, aromatischen oder N-, O- oder S-heteroaromatischen Mono-, Di-, Tri- oder Tetracarbonsäure mit 2 bis 22 Kohlenstoffatomen, die gegebenenfalls Keto- oder Hydroxygruppen enthält; oder der Rest der Aminoessigsäure, Dodecylmercapto propionsäure, 3,5 - Di - t - butyl - 4 - hydroxyphenyl - propionsäure, 2,2,6,6 - Tetramethyl - piperidin - 4 - carbonsäure, 3,8,8,10,10 - Pentamethyl - 9 - aza - 1,5 - dioxaspiro[5,5] - undecan - 3 - carbonsäure, Iminodiessigsäure, Thiodipropionsäure, Diglykolsäure, Dicarboxyethylpiperidin und Nitrilotriessigsäure ist;

$R_{13}$ ausgewählt ist aus der aus Wasserstoff; Alkyl- oder Alkoxygruppen mit 1 bis 18 Kohlenstoffatomen; Cycloalkyl- oder Cycloalkoxygruppen mit 3 bis 8 Kohlenstoffatomen; aromatischen, O-aromatischen oder araliphatischen und O-araliphatischen Gruppen mit 6 bis 18 Kohlenstoffatomen;

$$R_7\!-\!O\!-\!\underset{\underset{O}{\|}}{C}\!-\!(O)\!-;$$

einwertigen Oxysäuregruppen, abgeleitet von phosphorhaltigen Säuren, Kieselsäure oder Borsäure; und (4-Hydroxy-3,5-di-t-butylphenyl)-methyl bestehenden Gruppe;

m eine Zahl von 0 bis 10 ist;

n eine Zahl von 1 bis 4 ist;

p = 0, 1 oder 2 ist;

q eine Zahl von 0 bis 3 ist; und

r = 0 oder 1 ist.

2. Verbindungen nach Anspruch 1, worin m = 0 oder 1 ist.

3. Verbindungen nach irgendeinem der Ansprüche 1 bis 2, in welchen $R_4$ Wasserstoff ist oder $R_4$ und/oder $R_6$ eine aliphatische Gruppe mit 1 bis 18 Kohlenstoffatomen sind.

4. Verbindungen nach irgendeinem der Ansprüche 1 bis 3, worin $R_1$ Hydroxy ist und/oder $R_2$ Wasserstoff ist.

5. Verbindungen nach irgendeinem der Ansprüche 1 bis 4, worin

$R_1$

$$R_7\!-\!O\!-\!\underset{\underset{O}{\|}}{C}\!-\!O\!-,\ (R_7\!-\!O\!-\!\underset{\underset{O}{\|}}{C})_qR_{12}(\underset{\underset{O}{\|}}{C}\!-\!O)_n\!-,\ (R_7\!-\!O)_{2-n}\!-\!\underset{\underset{R_{13}}{|}}{\overset{\overset{(O)_n}{\|}}{P}}\!-\!(O)_n\!-\ \text{und}\ R_2\ \ -CH_2\!-\!\underset{\underset{R_4}{|}}{CH}\!-\!OR_{10}.$$

sind.

6. Verbindung nach Anspruch 1 mit der Struktur:

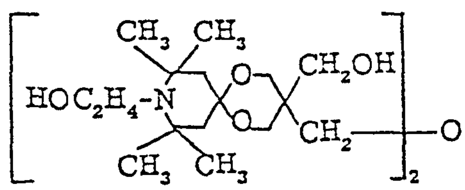

7. Eine Polyvinylchloridharz-Zusammensetzung mit verbesserter Beständigkeit gegen Zersetzung, insbesondere beim Erhitzen auf 177°C (350°F), die ein Polyvinylchloridharz, das mindestens teilweise aus der wiederkehrenden Gruppe

$$-\underset{\underset{Cl}{|}}{CH}\!-\!\underset{\underset{X}{|}}{\overset{\overset{X}{|}}{C}}-$$

gebildet wird und einen Chlorgehalt über 40% aufweist, worin X entweder Wasserstoff oder Chlor ist, und eine Verbindung gemäß irgendeinem der Ansprüche 1 bis 6 umfaßt.

8. Eine Olefinpolymerzusammensetzung mit verbesserter Beständigkeit gegen Zersetzung, die ein Olefinpolymer, ausgewählt aus der aus Polymeren von α-Olefinen mit 2 bis 6 Kohlenstoffatomen bestehenden Gruppe, und eine Verbindung gemäß irgendeinem der Ansprüche 1 bis 6 umfaßt.

9. Eine Acrylnitril-Butadien-Styrol-Terpolymer-Zusammensetzung mit verbesserter Beständigkeit gegen Zersetzung, die ein Acrylnitril-Butadien-Styrol-Terpolymer und eine Verbindung nach irgendeinem der Ansprüche 1 bis 6 umfaßt.

10. Eine Ethylen-Vinylacetat-Copolymer-Zusammensetzung mit verbesserter Beständigkeit gegen Zersetzung, die ein Ethylen-Vinylacetat-Copolymer und eine Verbindung nach irgendeinem der Ansprüche 1 bis 6 umfaßt.

11. Eine Polyurethanharz-Zusammensetzung mit verbesserter Beständigkeit gegen Zersetzung, die ein Polyurethanharz und eine Verbindung nach irgendeinem der Ansprüche 1 bis 6 umfaßt.

**Revendications**

1. Dérivés de spiro[2,2,6,6-tétraméthyl-pipéridine-4,2'-(1',3'-diox(ol)ane] ayant la formule suivant:

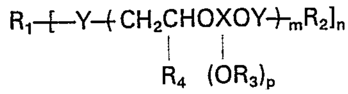 (I)

dans laquelle:

Y est

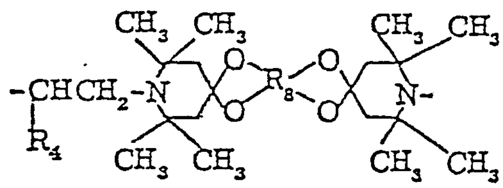

X est choisi dans la groupe comprenant les groupes alkylènes de 1 à 18 atomes de carbone; cycloalkylènes de 3 à 8 atomes de carbone, arylènes ou aralkylènes de 6 à 18 atomes de carbone; les groupes polyacyles

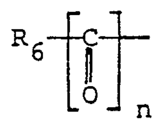

dans lesquels $n_1$ est 2, 3 ou 4; les groupes polycarbamoyles

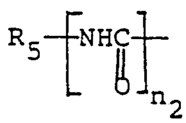

dans lesquels $n_2$ est 2, 3 ou 4;

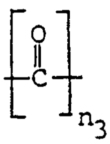

dans lesquels $n_3$ est 1 ou 2;

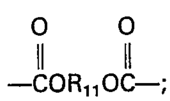

les rédidus de composés polyhydriques, diéthylèneglycol, triéthylèneglycol, thiodiéthylèneglycol, 1,4-cyclohexanediméthanol, bisphénol-A hydrogéné et isocyanurate de tris(2-hydroxyéthyle) et les groupes oxacides di- et trivalents dérivés des oxacides contenant du phosphore, de l'acide silicique ou de l'acide borique et les esters de ces acides ainsi que leurs dimères reliés par des alcools polyhydriques et des phénols;

$R_1$ est choisi dans le groupe comprenant les suivants: hydroxy,

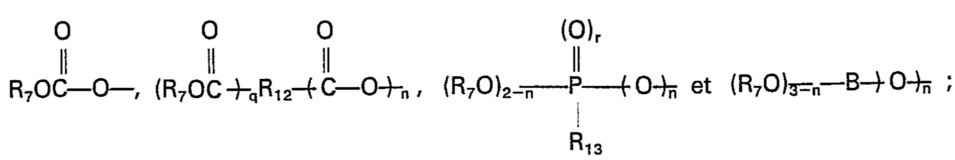

$R_2$ est choisi le groupe comprenant l'hydrogène et

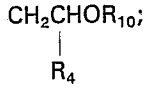

$R_3$ est choisi dans le groupe comprenant les groupes alkyles de 1 à 18 atomes de carbone; les groupes cycloalkyles de 3 à 8 atomes de carbone; les groupes aryles ou aralkyles de 6 à 18 atomes de carbone; et —X—O—$R_1$;

$R_4$ est choisi dans le groupe comprenant l'hydrogène; les groupes aliphatiques de 1 à 18 atomes de carbone; les groupes cycloaliphatiques de 3 à 8 atomes de carbone et les groupes aromatiques ou araliphatiques de 6 à 18 atomes de carbone;

$R_5$ est choisi dans le groupe comprenant les groupes aliphatiques de 1 à 18 atomes de carbone; les groupes cycloaliphatiques de 3 à 8 atomes de carbone et les groupes aromatiques ou araliphatiques de 6 à 18 atomes de carbone; et les résidus de diisocyanate de lysine, isocyanate de 3-isocyanatométhyl-3,5,5-triméthyl-cyclohexyle, bis-(isocyanatométhyl)-cyclohexane, isocyanate de 3-(2'-isocyanatocyclohexyl)-propyle et diisocyanate d'éther de diphényle;

$R_6$ est sélectionné dans le groupe comprenant les résidus d'acides di-, tri- ou tétracarboxyliques aliphatiques saturés ou insaturés, ces résidus ayant jusqu'à 18 atomes de carbone; les résidus d'acides di-, tri- ou tétracarboxyliques cycloaliphatiques saturés ou insaturés, ces résidus ayant jusqu'à 8 atomes de carbone; des résidus d'acides di-, tri- ou tétracarboxyliques aromatiques ou araliphatiques, ces résidus ayant jusqu'à 18 atomes de carbone, et les résidus des acides suivants: acide iminodiacétique, acide thiodipropionique, acide diglycolique, acide thiophène dicarboxylique, acide furanne dicarboxylique, dicarboxyéthylpipéridine et acide nitrilotriacétique;

$R_7$ est sélectionné dans le groupe comprenant les résidus d'alcools monohydriques de 1 à 18 atomes de carbone et des phénols éventuellement substitués par un (cyclo)alkyle ou un halogène ayant ensemble 6 à 50 atomes de carbone;

$R_8$ est choisi dans le groupe comprenant:

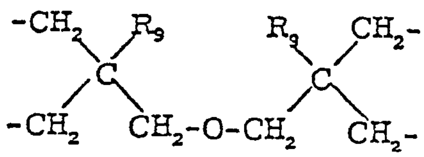
,
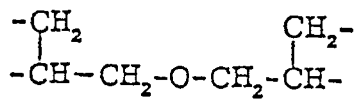
et

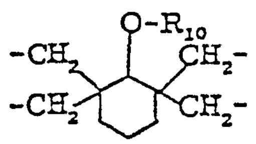

$R_9$ est choisi dans le groupe comprenant les groupes aliphatiques de 1 à 18 atomes de carbone, les groupes cycloaliphatiques de 3 à 8 atomes de carbone et —$CH_2OR_{10}$;

$R_{10}$ est choisi dans le groupe comprenant l'hydrogène,

$$-\overset{O}{\overset{\|}{C}}-R_{12}\left(COR_7\right)_{n+q-1},\quad -\overset{O}{\overset{\|}{C}}OR_7,\quad -\underset{R_{13}}{\underset{|}{\overset{(O)_r}{\overset{\|}{P}}}}-OR_7,\ \text{et}\ -B\left(OR_7\right)_2;$$

$R_{11}$ est choisi dans le groupe comprenant les groupes alkylènes et oxyalkylènes de 2 à 10 atomes de carbone et contenant de 0 à 5 groupes éther-oxydes; des groupes cycloalkylènes de 3 à 8 atomes de carbone et des groupes arylènes éventuellement substitués par un hydrocarbyle ayant ensemble 6 à 50 atomes de carbone, incluant les groupes phénylènes éventuellement substitués par un hydrocarbyle, et réunis par un atome d'oxygène ou de soufre ou par un groupe méthylène éventuellement substitué par un hydrocarbyle; isocyanurate; et les résidus de thiodiéthylèneglycol, 1,4 - phényldiméthanol, bisphénol-A hydrogéné, sulfure de bis - (2 - méthyl - 4 - hydroxy - 5 - t - butylbenzyle), 1,1,3 - tris - (2' - méthyl - 4' - hydroxy - 5' - t - butylphényl)butane, 2,2 - bis(3' - t - butyl - 4' - hydroxyphényl) - 4 - (3'',5'' - di - t - butyl - 4'' - hydroxyphényl) - butane et 2,2 - bis(2' - méthyl - 5' - t - butyl - 4' - hydroxyphényl) - 4 - (3'',5'' - di - t - butyl - 4'' - hydroxyphényl)butane;

$R_{12}$ est le résidu d'un acide mono-, di- tri- ou tétracarboxylique (cyclo)-aliphatique saturé ou insaturé, aromatique ou N-, O- ou S-hétéroaromatique ayant de 2 à 22 atomes de carbone et contenant éventuellement des groupes céto ou hydroxy; ou le résidu de l'acide aminoacétique, de l'acide dodécylmercaptopropionique, de l'acide 3,5 - di - t - butyl - 4 - hydroxyphénylpropionique, de l'acide 2,2,6,6 - tétraméthyl - pipéridine - 4 - carboxylique, de l'acide 3,8,8,10,10 - pentaméthyl - 9 - aza - 1,5 - dioxyaspiro[5,5]undécane - 3 - carboxylique, de l'acide iminodiacétique, de l'acide thiodipropionique, de l'acide diglycolique, de la dicarboxyéthylpipéridine et de l'acide nitriloltriacétique;

$R_{13}$ est choisi dans le groupe comprenant l'hydrogène, les groupes alkyles ou alcoxy de 1 à 18 atomes de carbone; les groupes cycloalkyles ou cycloalcoxy de 3 à 8 atomes de carbone; les groupes aromatiques, O-aromatiques ou araliphatiques et O-araliphatiques de 6 à 18 atomes de carbone,

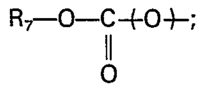

les groupes oxacides monovalents dérivés des acides contenant du phosphore, de l'acide silicique ou de l'acide borique et (4-hydroxy-3,5-di-tert-butylphényl)méthyle;

m est un nombre de zéro à 10;

n est un nombre de 1 à 4;

p est zéro, 1 ou 2;

q est un nombre de zéro à 3; et

R est zéro ou 1.

2. Composés selon la revendication 1, caractérisés en ce que m est zéro ou 1.

3. Composés selon la revendication 1 ou 2, caractérisés en ce que $R_4$ est l'hydrogène ou bien $R_4$ et/ou $R_6$ sont un groupe aliphatique de 1 à 18 atomes de carbone.

4. Composés selon l'une quelconque des revendications 1 à 3, caractérisés en ce que $R_1$ est l'hydroxy et/ou $R_2$ est l'hydrogène.

5. Composés selon l'une quelconque des revendications 1 à 4, caractérisés en ce que

$R_1$ is

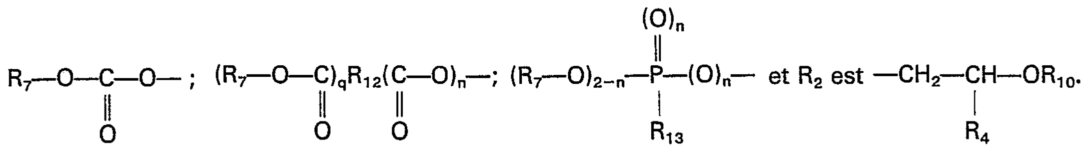

; ; et $R_2$ est

6. Composé selon la revendication 1 ayant la structure:

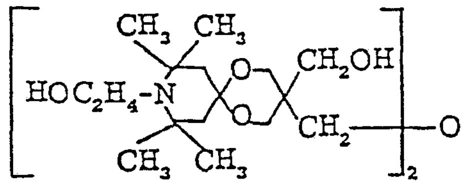

7. Une composition de résine de chlorure de polyvinyle ayant une résistance améliorée à la détérioration, spécialement lorsqu'elle est chauffée à 177°C, caractérisée en ce qu'elle comprend une résine de chlorure de polyvinyle formée au moins en partie du groupe de récurrence:

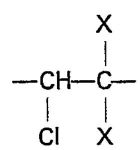

et ayant une teneur en chlore supérieure à 40%, où X est l'hydrogène ou le chlore, et un composé selon l'une quelconque des revendications 1 à 6.

8. Une composition de polymère d'oléfine ayant une résistance améliorée à la détérioration, caractérisée en ce qu'elle comprend un polymère d'oléfine choisi dans le groupe des polymères d'alpha-oléfines ayant de 2 à 6 atomes de carbone, et un composé selon l'une quelconque des revendications 1 à 6.

9. Une composition de terpolymère d'acrylontrilebutadiène-styrène ayant une résistance améliorée à la détérioration, caractérisée en ce qu'elle contient un terpolymère d'acrylonitrile-butadiène-styrène et un comosé selon l'une quelconque des revendications 1 à 6.

10. Une composition de copolymère d'éthylène-acétate de vinyle ayant une résistance améliorée à la détérioration, caractérisée en ce qu'elle comprend un copolymère d'éthylène-acétate de vinyle et un composé selon l'une quelconque des revendications 1 à 6.

11. Une composition de résine de polyuréthanne ayant une résistance améliorée à la détérioration, caractérisée en ce qu'elle comprend une résine de polyuréthanne et un composé selon l'une quelconque des revendications 1 à 6.